# EUROPEAN PATENT APPLICATION

(11) **EP 4 176 875 A1**
(43) Date of publication of application: **10.05.2023**
(21) Application number: 21306547.7
(22) Date of filing: 04.11.2021
(51) Int. Cl.: A61K 31/404, A61P 11/00, A61P 31/14

(54) **PHARMACEUTICAL COMPOSITION, ITS USE AS A DRUG AND NEW COMPOUNDS, ESPECIALLY FOR TREATING SARS-COV-2 INFECTION**

(71) Applicant: Université de Bordeaux, 33000 Bordeaux (FR); Centre National de la Recherche Scientifique, 75016 Paris (FR); Institut National de la Santé et de la Recherche Médicale, 75013 Paris (FR); Université Paris Cité, 75006 Paris (FR); Université d'Orléans, 45067 Orléans Cedex 2 (FR); École Normale Supérieure Paris-Saclay, 91190 Gif-sur-Yvette (FR); Université Paris-Saclay, 91190 Gif-sur-Yvette (FR); REQUIMTE - Rede de Químíca e Tecnologia - Associação, 4050-453 Porto (PT)
(72) Inventor: PARISSI, Vincent, 33300 Bordeaux (FR); SOUSA, Sergio, 4440-752 Valongo (PT); LAPAILLERIE, Delphine, 33600 Pessac (FR); DELELIS, Olivier, 78220 Viroflay (FR); MEERTENS, Laurent, 95170 Deuil La Barre (FR); GALLOIS-MONTBRUN, Sarah, 92370 Chaville (FR); ROUTIER, Sylvain, 45510 Tigy (FR)
(74) Representative: Novagraaf Technologies

(57) **Abstract**

The present invention relates to a compound comprising a compound having formula (I) below: 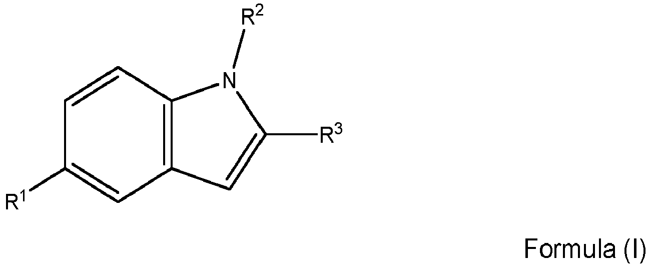 wherein R¹, R² and R³ are as defined, or a pharmaceutically acceptable salt thereof, for use in the prevention or in the treatment of an infection.

The invention also relates to a pharmaceutical composition comprising the compound, for use in the prevention or in the treatment of an infection.

## Description

### Technical field

The present invention refers to a compound or a pharmaceutically acceptable salt thereof, for use in the prevention or the treatment of an infection, especially for use in the prevention or in the treatment of SARS-CoV-2 infection.

Therefore, the present invention has utility in medical field, especially in the field of treatment of infection diseases.

In the description below, the references into brackets ([]) refer to the listing of references situated at the end of the text.

### Background of the Invention

Severe Acute Respiratory Syndrome-coronavirus 2 (SARS-CoV-2) is the positive-strand RNA virus causing agent of severe respiratory syndrome in humans (COVID-19).

Viral entry into cells requires the interaction between the spike S protein of the virus and the Angiotensin-Converting enzyme 2 ACE2 at the surface of the cell membrane. ACE2 has been reported as receptor for SARS-CoV-2 in lung as previously shown for SARS-CoV responsible for the first SARS outbreak in 2003. ACE2 is expressed in human airway epithelia, lung parenchyma, vascular endothelia, kidney cells, and small intestine cells but only weakly detected in the brain. Thus, converging data from the literature indicate the S/ACE2 interaction remains the central entry way for SARS-CoV-2 virus. Being the first contact point between the virus and the cell, the S/ACE2 complex constitute an attractive target for blocking the very early events of the viral infection.

While several vaccines have been developed since the beginning of the pandemic, no efficient drugs have been identified as having strong effect on viral replication usable in clinic, partly due to the lack of understanding of viral replication processes.

Therefore, a better understanding of the replication processes and the search for new antiviral approaches remain crucial.

Thus, a need exists of drugs allowing to treat, or at least decrease the symptoms due to SARS-CoV-2 infection. The present invention fulfills these and other needs.

### Description of the invention

The Applicant surprisingly found that compounds derived from indole have the capability to block the infectivity of lentiviral vectors pseudotyped with the SARS-CoV-2 S protein as well as wild type and variants SARS-CoV-2 circulating strains in various human cell lines, including pulmonary cells naturally susceptible to the infection.

AlphaLISA and biolayer interferometry confirmed a direct inhibitory effect of the compounds on the S/ACE2 association.

Thus, after extensive works, the Applicant found novel drugs capable to specifically block the SARS-CoV-2 entry step by impairing the ACE2/S interaction leading to inhibition of the viral SARS-CoV-2 replication in naturally susceptible cells.

Accordingly, in a first aspect, the present invention provides a compound comprising a compound having formula (I) below: 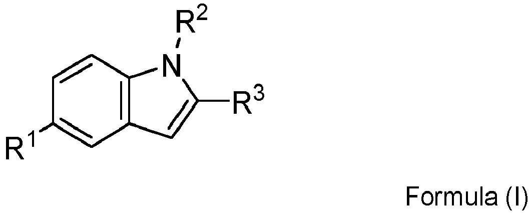 wherein:
- R¹ is chosen from the group comprising: 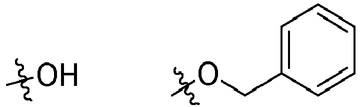 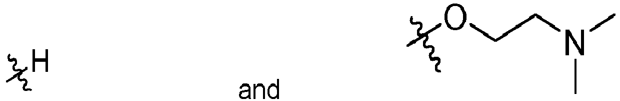
- R² is chosen from the group comprising: 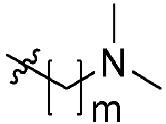 m being an integer from 2 to 6, 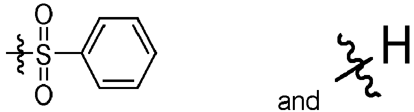
- R³ is chosen from the group comprising: 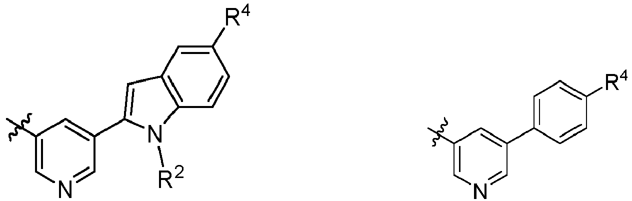 and 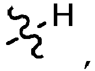 in which R² is defined as above,
   and R⁴ is chosen among : 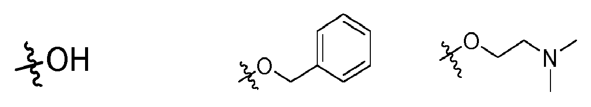 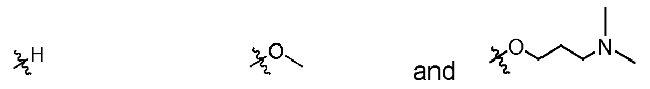 or a pharmaceutically acceptable salt thereof,
for use in the prevention or in the treatment of an infection.

The term "pharmaceutically acceptable salt" is meant to include any salt commonly used in the pharmaceutical field and that is adapted to indole derivatives used in the invention according to the general technical knowledge of the skilled person. It can be for example hydrochloride, acetate, phosphate or methanesulfonate, fumarate and oxalate.

The compounds may be prepared by methods known in the art, for example in document Jacquemard et al. (Synthesis of 3,5-bis(2-indolyl)pyridine and 3-[(2-indolyl)-5-phenyl]pyridine derivatives as CDK inhibitors and cytotoxic agents. (2008) Bioorg. Med. Chem., 16, 4932-4953 ([1])) and in document Mahboobi et al. (Bis(1H-2-indolyl)methanones as a novel class of inhibitors of the platelet-derived growth factor receptor kinase; J. Med. Chem 2002 45 1002-1018 ([13])).

According to the invention, the infection prevented or treated by the compound may be for example an infection caused by a virus for which viral entry into cells requires the interaction between the spike S protein of the virus and the Angiotensin-Converting enzyme 2 ACE2 at the surface of the cell membrane. Advantageously, the compound may inhibit the interaction between Spike and ACE2 proteins.

The virus may be, for example, selected from the Coronaviridae family. Viruses of the Coronaviridae family may be for example selected from the group comprising Coronavirus SARS-CoV-2, Coronavirus SARS, Coronavirus 229E, Coronavirus NL63, Coronavirus OC43, Coronavirus HKU1 and Coronavirus MERS-CoV. In a preferred embodiment of the invention, the viral infection is a SARS-CoV-2 infection. The SARS-CoV-2 may be the original strain appeared in 2019, or variants appeared from then, including alpha variant, beta variant, gamma variant and delta variant.

Advantageously, in the compound for use of the invention:
- R² may be chosen from the group comprising: 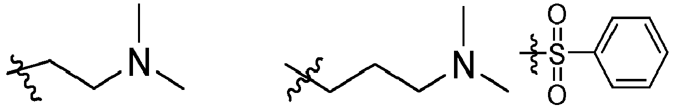 and 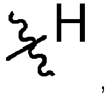 and
- R³ may be chosen from the group comprising:

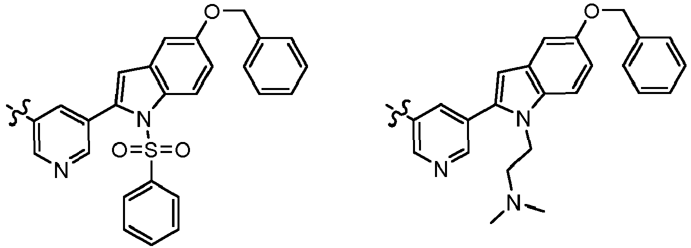

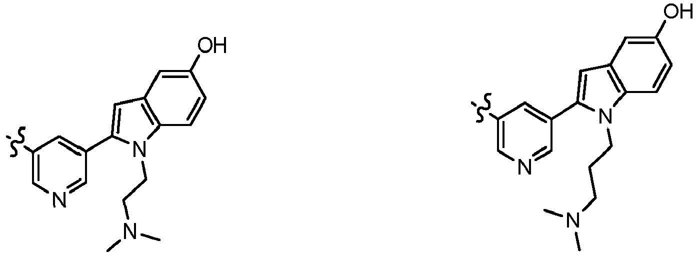

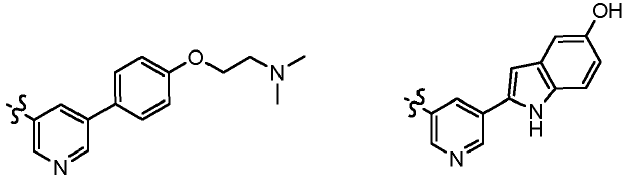

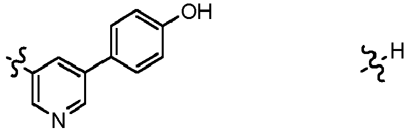

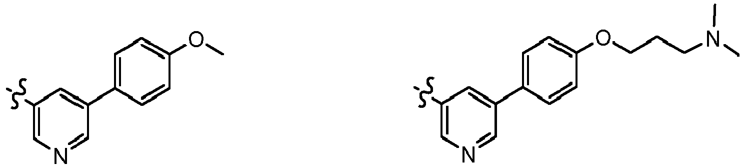

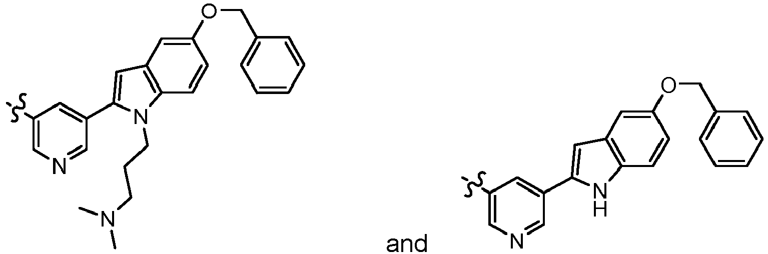

Advantageously, particular compounds for use according to the invention may be chosen among: 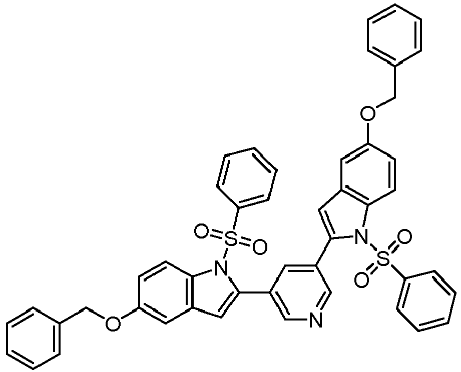 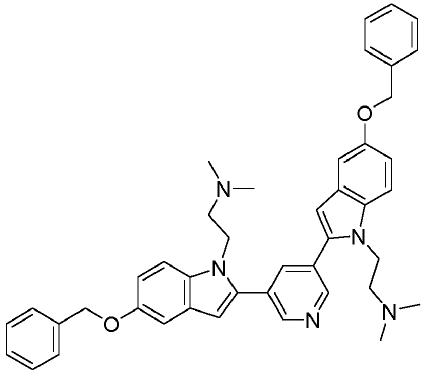 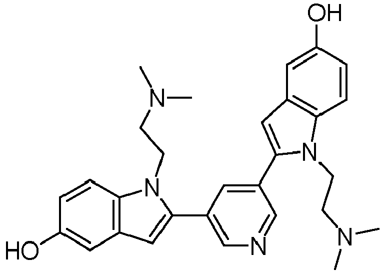 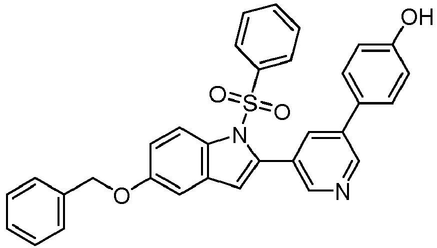 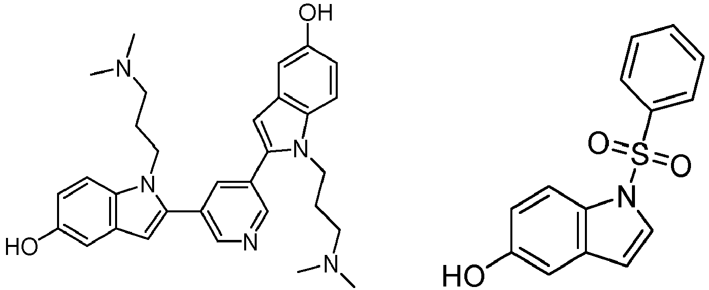 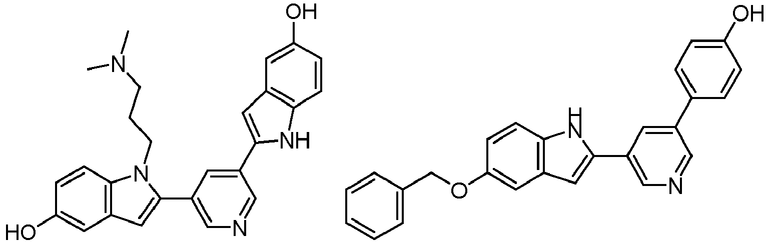 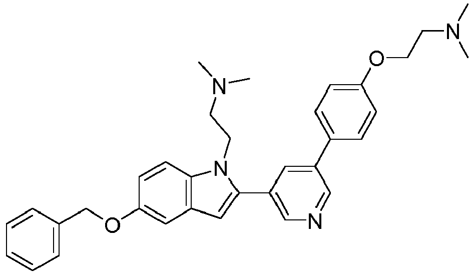 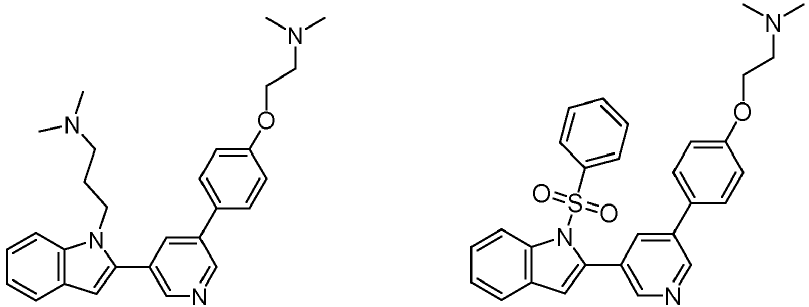 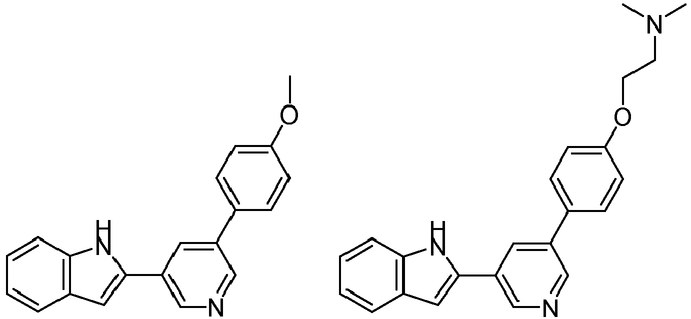 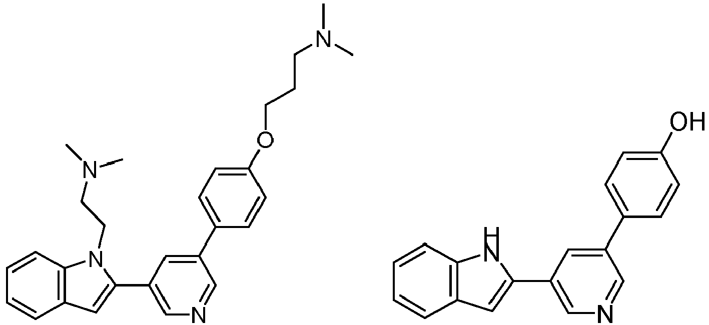 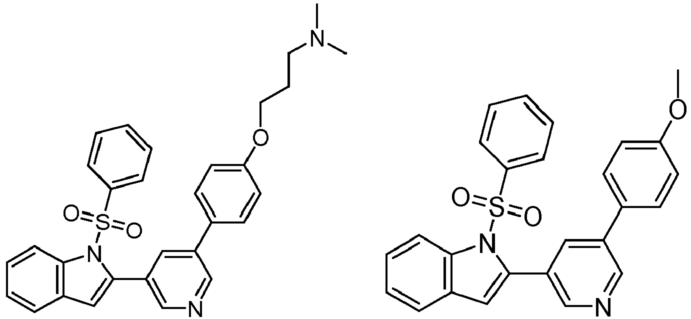 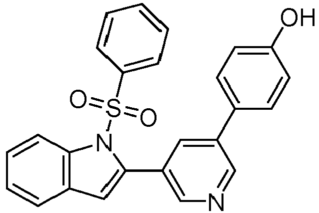 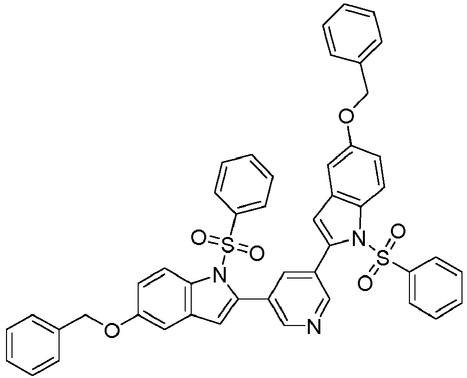 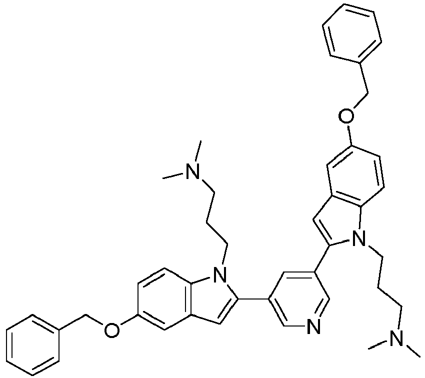 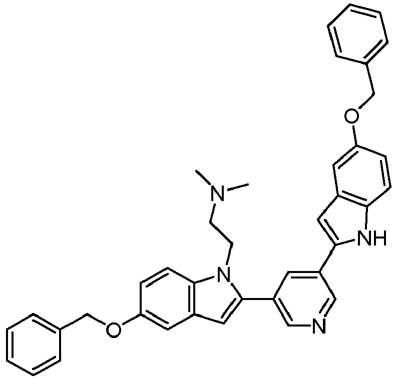 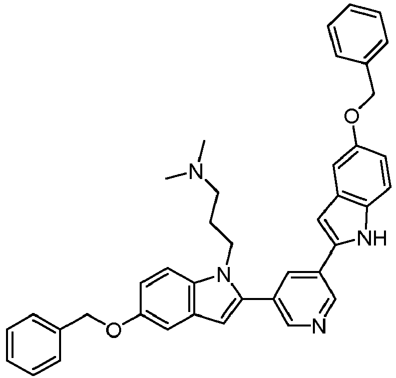 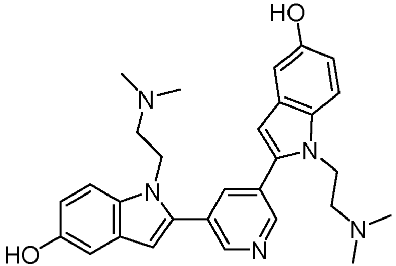 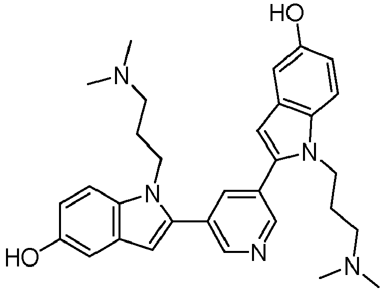 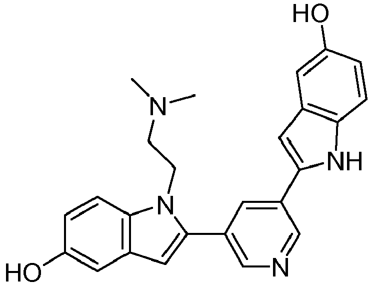 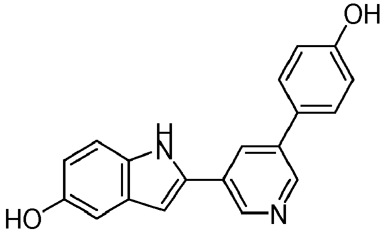 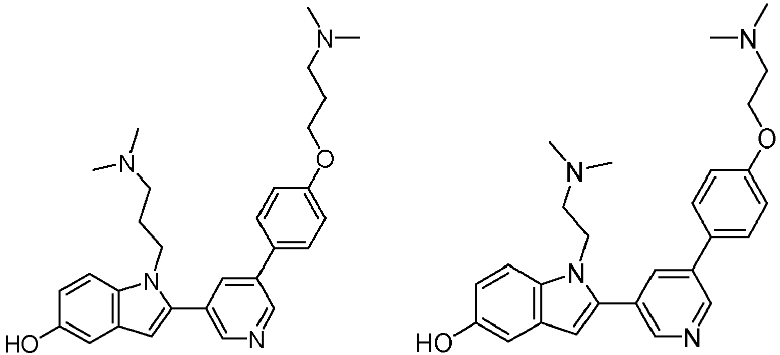 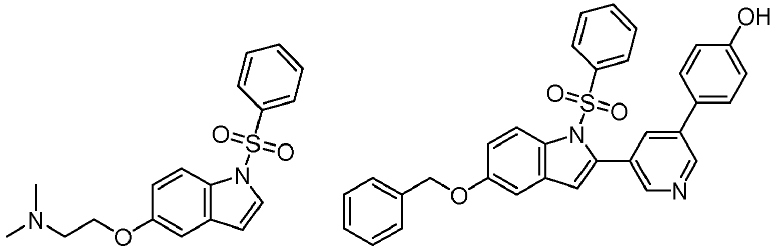 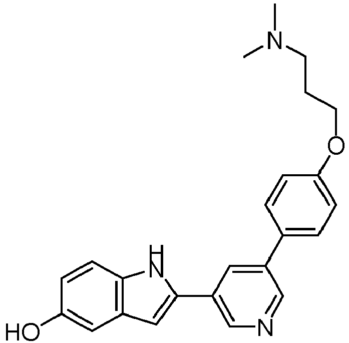 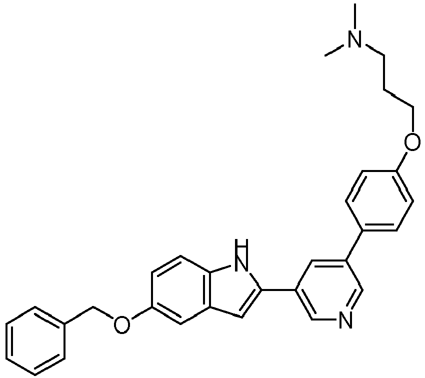 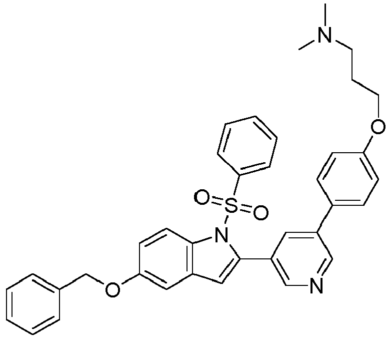 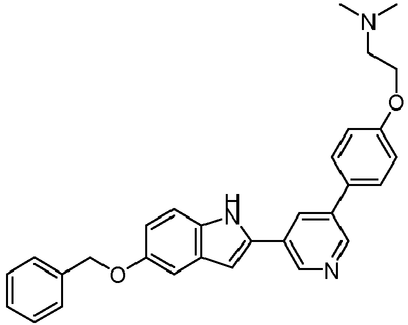 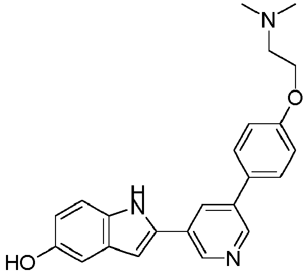 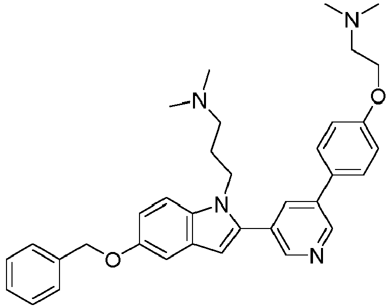 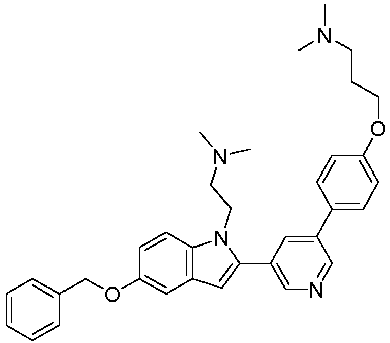 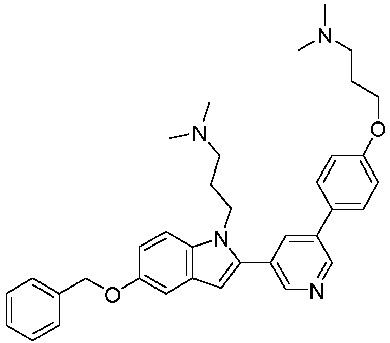 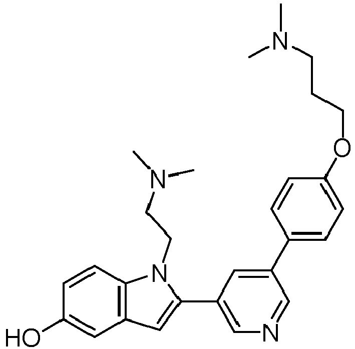 and 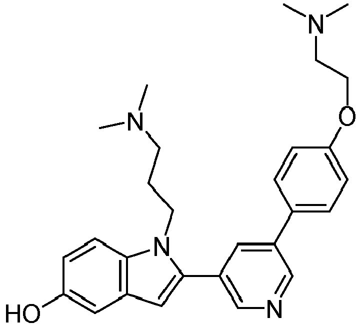 or a pharmaceutically acceptable salt thereof.

According to the use of the invention, the compound may be in a enteral, parenteral or topical form. The compound may be for example in a form for oral administration, for example selected from the group comprising an oral solution, a syrup, an oral suspension, an emulsion and oral drops. Alternatively, it may be in an oral effervescent dosage form, selected from the group comprising tablets, granules, powders. Alternatively, it may be in the form of an oral powder or a multiparticulate system, for example selected from the group comprising beads, granules, mini tablets and micro granules. Alternatively, it may be in the form of an orodispersible dosage form, selected from the group comprising orodispersible tablets, lyophilised wafers, thin films, a chewable tablet, a tablet and a capsule, a medical chewing gum. Alternatively, it may be in a form for buccal and sublingual routes, for example selected from the group comprising buccal or sublingual tablets, muco adhesive preparation, lozenges, oro-mucosal drops and sprays. Alternatively, it may be in a form for topical-transdermal administration, for example selected from the group comprising ointments, cream, gel, lotion, patch and foam. Alternatively, it may be in a form for a nasal administration, for example selected from the group comprising nasal drops, nasal spray, nasal powder. Alternatively, it may be in a form for a rectal administration, for example suppository or hard gelatin capsule. Alternatively, it may be in a form for parenteral administration, for example subcutaneous, intramuscular, intravenous administration.

Another object of the invention relates to a pharmaceutical composition comprising a compound as defined above, for use in the prevention or in the treatment of an infection as defined above.

The term "pharmaceutical composition" refers herein to a composition comprising at least one active drug and at least one pharmaceutically acceptable excipient, that can be administered to animals and to human beings for the treatment of the disorders and diseases as mentioned above.

According to the invention, one active drug is the compound as defined above. Advantageously, the pharmaceutical composition may comprise at least one other active drug, for example chosen among dexamethasone, remdesivir, azithromycin. The pharmaceutically acceptable excipient may be any substance adapted to be formulated alongside the active ingredient (s) of the pharmaceutical composition of the invention. It may be for example selected among antiadherents, binders, coatings, colours, disintegrants, flavors, glidants, lubricants, preservatives, sorbents, sweeteners, surfactants, lipids, and vehicles.

The pharmaceutical composition may be in any pharmaceutical form, i.e. enteral, parenteral or topical. It may be a medicament for oral administration, for example selected from the group comprising a solution, a syrup, a suspension, an emulsion and oral drops. Alternatively, it may be a medicament in the form of an oral effervescent dosage form, selected from the group comprising tablets, granules, powders. Alternatively, it may be an oral powder or a multiparticulate system, in a form selected from the group comprising beads, granules, mini tablets and micro granules. Alternatively, it may be a medicament in the form of an orodispersible dosage form, selected from the group comprising orodispersible tablets, lyophilised wafers, thin films, a chewable tablet, a tablet and a capsule, a medical chewing gum. Alternatively, it may be a medicament for buccal, intratracheal and sublingual routes, for example selected from the group comprising buccal or sublingual tablets, muco adhesive preparation, lozenges, oro-mucosal drops and sprays. Alternatively, it may be a medicament for topical-transdermal or local administration, for example selected from the group comprising ointments, cream, gel, lotion, patch and foam. Alternatively, it may be a medicament for nasal administration, for example selected from the group comprising nasal drops, nasal spray, nasal powder. Alternatively, it may be a medicament for rectal administration, for example suppository or hard gelatin capsule. Alternatively, it may be a medicament for parenteral administration, for example subcutaneous, intramuscular, intravenous administration.

The pharmaceutical composition of the invention may be prepared using conventional techniques known by the skilled person. For example, the bulk drug substance may be dissolved in a suitable solvent in the presence of one or more of the excipients described above, and mixed.

Advantageously, the pharmaceutical composition may allow delivery of a pharmaceutically acceptable and efficient dose of the compound, for the prevention or the treatment of an infection as defined above. For example, the pharmaceutical composition may comprise a dose of compound of 1 to 50 mg/kg of body weight per day, preferably a dose of 1 to 20 mg/kg of body weight per day. The administration may be carried out with one dose or with a plurality of doses per day.

This invention is further illustrated by the following examples with regard to the annexed drawings that should not be construed as limiting.

### Brief description of the figures

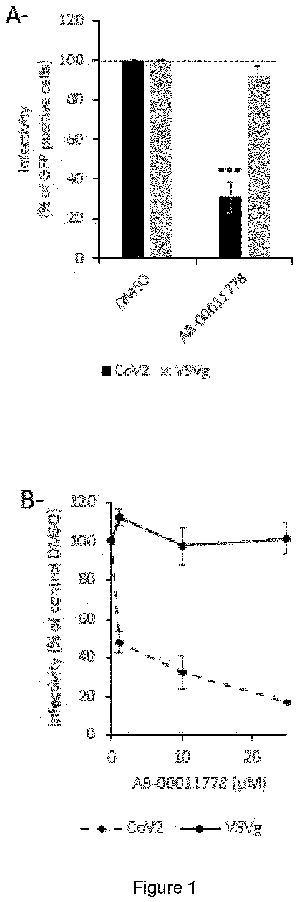

- Figure 1: represents the characterization of AB-00011778 compound, using increasing concentration of drugs. Infectivity (% of GFP positive cells) of COV2 or VSVg in presence of AB-00011778 compound or DMSO (A). Infectivity (% of control DMSO) of COV2 or VSVg in presence of AB-00011778 compound (B). Data are shown as means of at least three independent experiment ±standard deviation. ***p<0.001, **p<0.01.

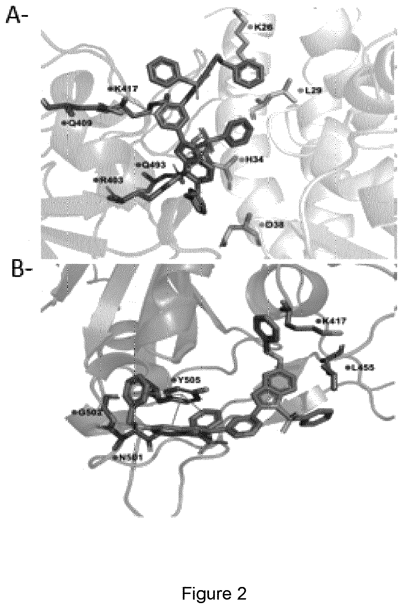

- Figure 2: represents molecular docking of AB-00011778 compound to the spike/ACE2 complex and to the spike protein alone. Data were obtained from the predicted binding poses of the compounds to the S-RDB and to S-RDB/ACE2 interface. The interacting amino acid residues have been highlighted. (A) AB-00011778 compound bound to the spike/ACE2 complex; (B) AB-00011778 bound to the spike alone.

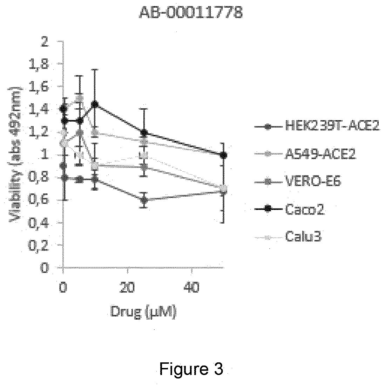

- Figure 3: represents the cytotoxicity of AB-00011778 compound in various cell lines (HEK293T-ACE2, A549-ACE2, VERO-E6, Caco2, Calu3). The viability of the cells (492nm absorbance in MTT assay) was plotted against the increasing concentration of drugs. Data are shown as means of at three independent experiments. Data are shown as means of at least three independent experiments ± (SD).

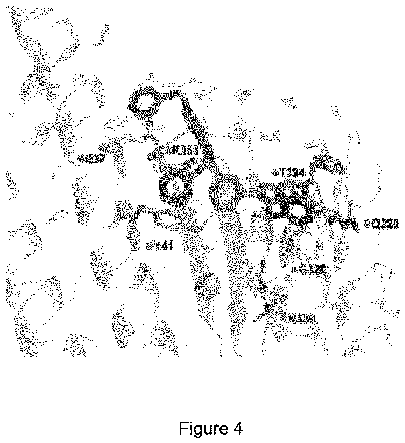

- Figure 4: represents molecular docking of AB-00011778 bound to ACE2 alone. Data were obtained from the predicted binding poses of the compounds to the ACE2 around the interface region. The interacting amino acid residues have been highlighted.

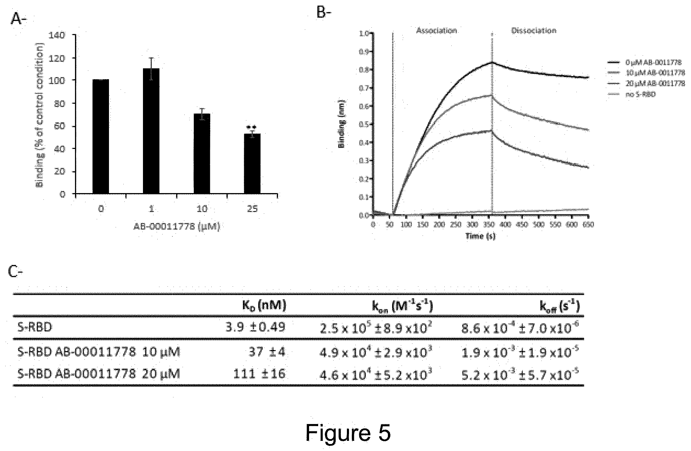

- Figure 5: represents the effect of AB-00011778 compound on the *in vitro* S-RBD/ACE2 interaction using AlphaLISA and Bio-Layer Interferometry (BLI) technologies. SARS-CoV-2 S-RBD(His)₆ and human ACE2-Biot interaction have been first monitored by AlphaLISA (A) using 3 nM of each protein. Increasing concentrations of AB-00011778 drug were incubated for 30 min with S-RBD(His)₆ before being mixed with ACE2-Biot for 2h. The microplate was read after 2 h incubation with anti-6X His acceptor and streptavidin donor beads. Data obtained with the compounds were compared to DMSO control and are reported as mean of percentage of control condition from two to three independent experiments in duplicate ± standard deviation. **p<0.01. The effect of the drug on the binding kinetics of S-RBD to biotinylated ACE2 immobilized on streptavidin biosensor was also performed using BLI experiments (B). Baseline with reaction buffer was measured for 60 s. For the association step (300 s), each loaded biosensor was dipped into 50 nM of S-RBD(His)₆ preincubated for 15 min with increasing concentrations of AB-00011778 compound. The dissociation step was subsequently measured for 300 s. Sensorgrams curves shown in (B) were plotted using Prism 5.0 software (Graphpad Software, La Jolla, CA). The association and dissociation experimental curves were local fitted using a 1:1 binding model with the Blitz pro 1.1 software. Kinetic binding parameters (kₒₙ, k_{off}) and the equilibrium dissociation constant (K_{D}) were determined as means of two to three independent experiments and reported in (D). Data are shown as means of two to three independent experiment ±standard deviation. ***p<0.001, **p<0.01.

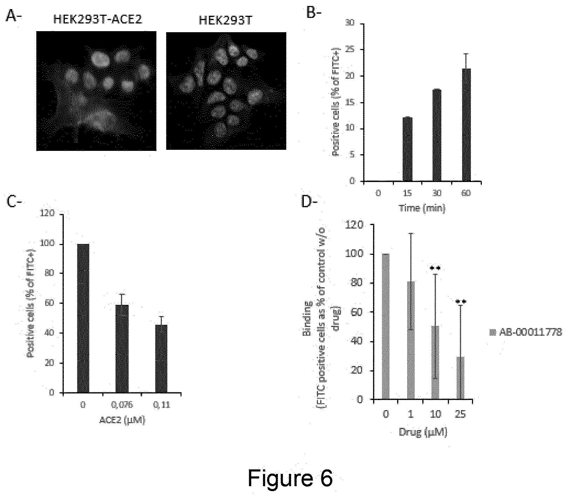

- Figure 6: represents the effect of AB-00011778 compound on the S-RBD/ACE2 interaction in cellular context. The SARS-CoV-2 S-RBD fragment was added to either HEK293T or HEK293-ACE2 cells for 0-60 minutes and immunofluorescence staining was performed using anti-(His)₆ antibody and secondary antibody coupled to Alexa-Fluo 488. Cells were observed by epifluorescence microscopy **(A).** Cells were also analyzed using flow cytometry to detect the percentage of positive cells for FITC signal during time **(B).** Increasing concentrations of either soluble ACE2 (C) or drug **(D)** were added with the RBD. Data are reported as mean of percentage of positive cells from at least three independent experiments. **p<0.05.

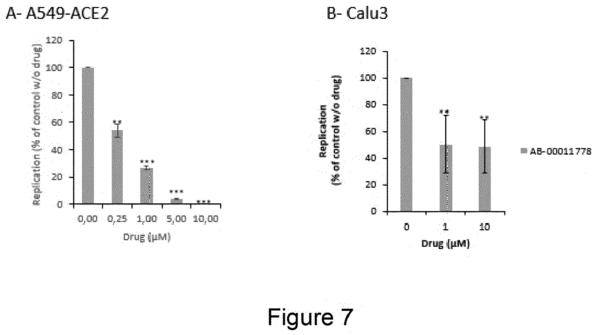

- Figure 7: represents effect of the AB-00011778 compound on SARS-CoV-2 replication in human pulmonary cells. A549-ACE2 (A), or Calu3 (B) cells were incubated with the SARS-CoV-2 reference strain (MOI=1) and increasing concentrations of the drug. The replication was evaluated by the quantification of the viral genome copies after 24 hours. Data are reported as means from 3-4 independent experiments and expressed as a percentage of control w/o drug.

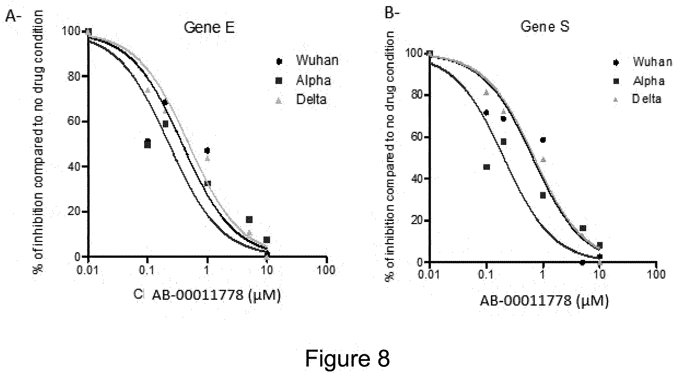

- Figure 8: represents the effect of the AB-00011778 on SARS-CoV-2 Delta and Alpa replication in human pulmonary cells. A549-ACE2-TMPRS2 cells were incubated with the SARS-CoV-2 Wuhan, Alpha or Delta strain (MOI=1) and increasing concentrations of drugs. The replication was evaluated by the quantification of the viral gene E (A) and S (B) genome copies of normalized to Actin RNA after 24 hours. A representative experiment is reported in the figure.

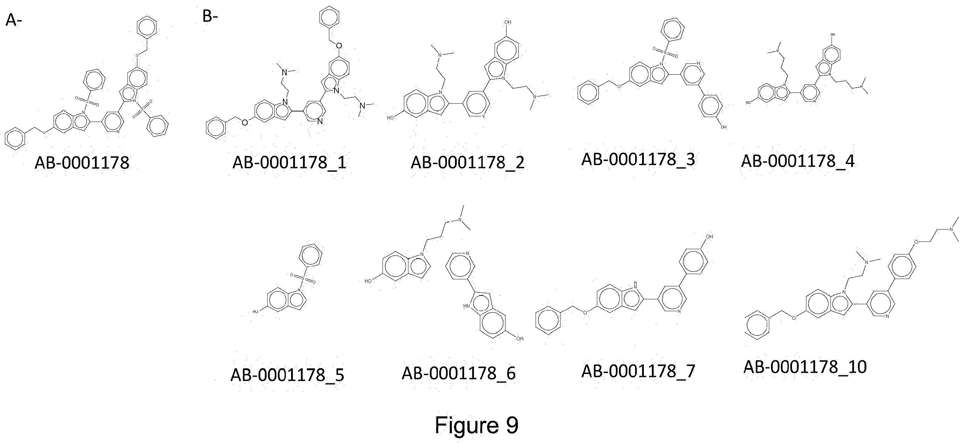

- Figure 9: represents chemical derivatives from the AB-0001178 compounds. The structure of the initially selected drugs is reported in (A). Several analogs have been designed and synthetized on the basis of the docking calculation performed in Figure 2.

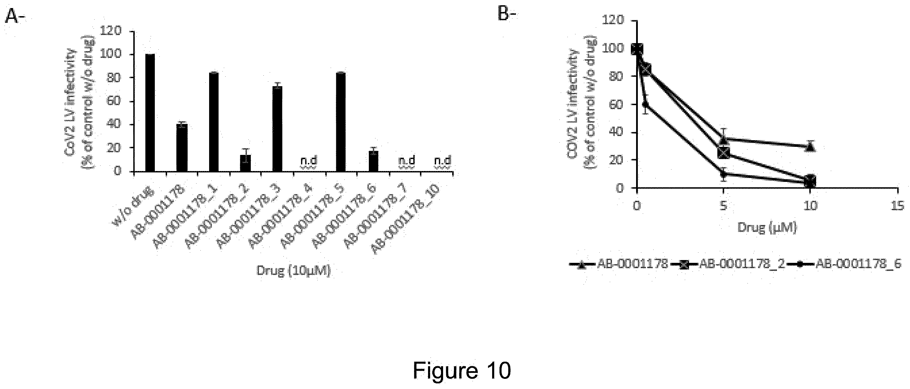

- Figure 10: represents Effect of the AB-0001178 and its derivatives on SARS-CoV-2 pseudotyped lenviruses in HEK-293T cells. 10µM concentration of the AB-0001178 derivatives (A) and increasing concentrations (0-10 µl) of AB-0001178, AB-0001178_2 and AD-0001178_6 (B) were added to an infectivity assay using SARS-CoV-2 pseudotyped lentiviral vector and HEK293T-Ace2 cells. Data are reported as mean of three experiments ±SD.

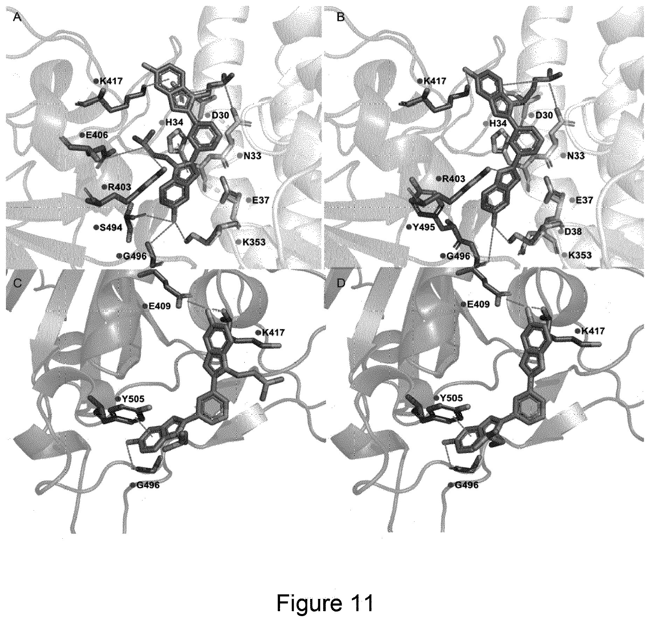

- Figure 11: represents interactions of the Analogues. (A) AB-00011778_2 with S-RDB/ACE2; (B) AB-00011778_6 with S-RDB/ACE2; (C) AB-00011778_2 with S-RDB; (D) AB-00011778_6 with S-RDB.

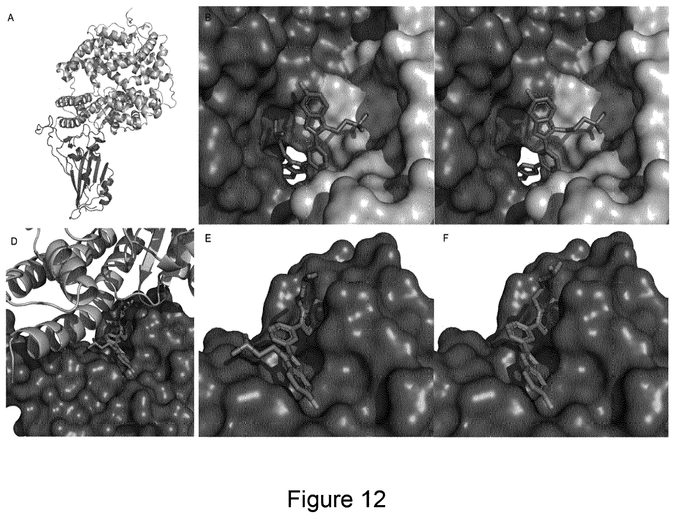

- Figure 12: represents the analysis of the Interactions of AB-00011778_2 and AB-00011778_6 to the spike/ACE2 complex and to the spike protein alone. (A) Detail on the S-RDB/ACE2 interaction; (B) AB-00011778_2 bound to the spike/ACE2 complex; (C) AB-00011778_6 bound to the spike/ACE2 complex; (D) Comparison of the crystallographic position of ACE2 vs the position occupied by AB-00011778_2 and AB-00011778_6 when bound to the spike protein alone; (E) AB-00011778_2 bound to the spike alone; (F) AB-00011778_6 bound to the spike alone.

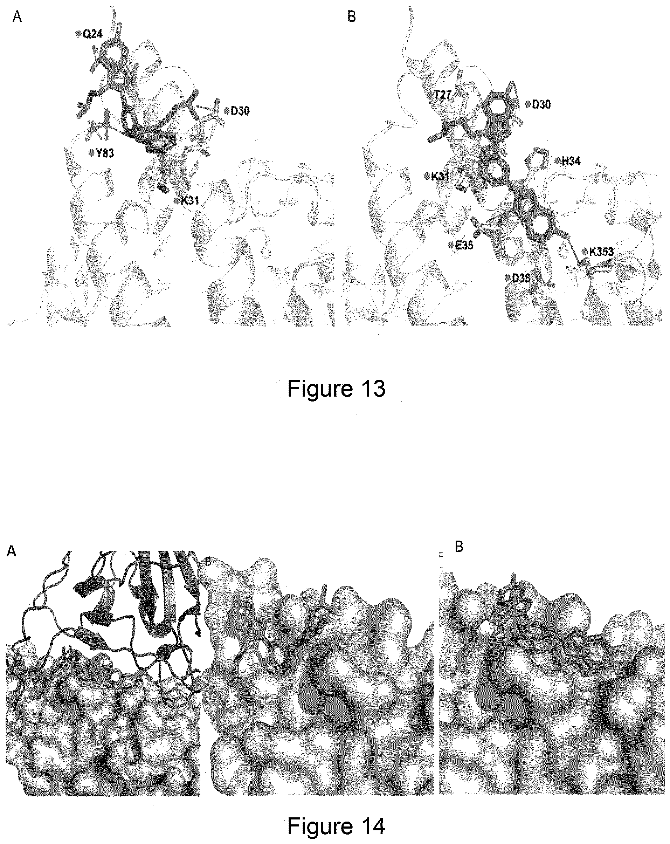

- Figure 13: represents interactions of the Analogues. (A) AB-00011778_2 with ACE2 alone; (B)AB-00011778_6 with with ACE2 alone.
- Figure 14: represents (A) Comparison of the crystallographic position of S-RDB vs the position occupied by AB-00011778_2 and AB-00011778_6 when bound to ACE2 alone; (B) AB-00011778_2 bound to ACE2 alone; (C) AB-00011778_6 bound to ACE2 alone.

### Examples

### Example 2: effects of indole derivatives as defined in the invention as new antiviral drugs targeting multiple SARS-CoV S/ACE2 interfaces

### In cellulo screen for specific S/ACE2-mediated SARS-CoV-2 entry pathway.

AB-00011778 compound of formula: 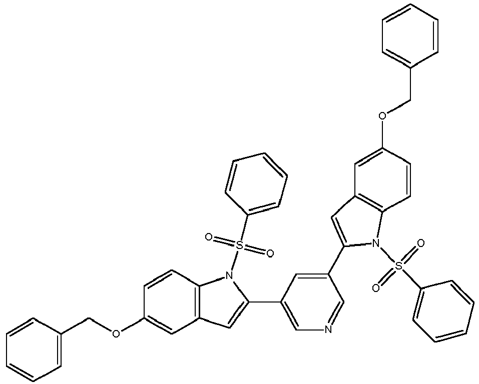 was selected and first tested on the lentiviral vectors-based cellular approach previously set up in the laboratory (Lapaillerie et al. (2021) In Silico, In Vitro and In Cellulo Models for Monitoring SARS-CoV-2 Spike/Human ACE2 Complex, Viral Entry and Cell Fusion. Viruses, 13 ([2])). Briefly, lentiviral vectors pseudotyped with the SARS-CoV-2 Spike protein (CoV-2 LV) have been produced from HEK293T cells transfected with three plasmids: a plasmid encoding a lentiviral backbone (LV44: pRRLSIN-PPT-hPGK-eGFP-WPRE) expressing a marker protein, the HDM_IDTSpike_fixK plasmid expressing the Spike protein (kind gift from the Bloom laboratory (Crawford et al. (2020) Protocol and Reagents for Pseudotyping Lentiviral Particles with SARS-CoV-2 Spike Protein for Neutralization Assays. Viruses, 12 ([4])) and the psPAX2 plasmid expressing the other HIV proteins needed for virion formation (Tat, Gag-Pol, and Rev). HEK293T-ACE2 cells containing 9 copies of the *ACE2* gene were generated by lentiviral vector insertion of the human *ACE2* gene in their genome ([2]). The CoV-2 LVs were shown to transduce efficiently only the 293T-ACE2 cells in contrast to the original 293T cells leading to the integration and expression of the *eGFP* gene encoded by the LV. This confirms that the transduction efficiency monitored by the percentage of eGFP positive cells relies on the early S/ACE2 interaction. On the contrary, typical VSVg pseudotyped LV were able to transduce both 293T and 293T-ACE2 cells and will serve as control for the next selection of drugs specific for S/ACE2 entry. Inhibition competition experiments using the soluble ACE2 protein fully validated the S/ACE2 dependency of the LV CoV-2 infectivity while the LV VSVg was not affected by the protein (data not shown) ([2]).

AB-00011778 compound was thus tested on both VSVg and SARS-CoV-2 LVs infectivity to determine its possible effect on the S/ACE2 mediated entry pathway. A first screen using 10 µM concentration of drug showed significant inhibitory effect for some molecules on CoV-2 LVs without any effect on VSVg LVs (see Figure 1A). AB-00011778 was found efficient by inducing a 40 to 80% decrease of the viral infectivity. Further accurate analysis of the inhibitory effect of the compound using increasing concentrations confirmed their specific capability of inhibiting the CoV-2 LVs infectivity (Figure 1B) without significant effect on the VSVg LVs within the 0-20µM range. The cytotoxicity of the molecule determined in different cell lines including HEK-293T, A549, Calu3 and Caco2 cell lines using MTT assay showed no or poor cytotoxicity within the 0-50µM range (see Figure 3).

Taken together our data strongly suggested that AB00011778 compound interfered with the early S/ACE2 mediated SARS-CoV-2 entry process. To confirm this, we next investigated whether this inhibitory effect could be due to direct effect of the drugs on the physical interaction between S and ACE2 using molecular docking and biochemical approaches.

### Molecular docking analysis of the drugs interactions with S and ACE2

To better understand the binding of AB-00011778 compound to the target protein, molecular docking studies were performed. Figure 2 displays results from the predicted binding poses to the S-RDB/ACE2 interface and to the S-RBD, highlighting the interacting amino acid residues.

The docking of AB-00011778 compound to the S-RDB/ACE2 interface (Figure 2A) resulted in a score of 92.05. S-RDB residues Arg403, Gln409 and Lys417 establish strong hydrogen bonds with this molecule. Arg403 interacts with one oxygen from one of the sulfonyl groups with a distance of 3.00 Å. Gln409 and Lys417 interact with the nitrogen from the central pyridine with distances of 3.78 Å and 3.55 Å, respectively. In addition, ACE2 His34 interacts with the indole group via π-π stacking interactions. ACE2 residues Lys26, Leu29, and Asp38, and S-RDB residues Lys417 and Gln493 are also involved in hydrophobic interactions with AB-00011778 compound. The interactions of the AB-00011778 compound with the amino acid residues ACE2 Asp38 and with S-RDB Lys417 and Gln493 are of special relevance. In fact, previous MD studies of the S-RDB/ACE2 interface ([2]) have shown that Asp38 from ACE2 establishes important hydrogen bonds with S-RDB Tyr449 and Gln498; S-RDB Lys417 was shown to be involved in very stable hydrogen bonds and charged interactions with Asp30 from ACE2, while S-RDB Gln493 formed important hydrogen bonds with ACE-2 Glu35. The disruption of these interactions caused by AB-00011778 compound binding should lead to the blocking the S/ACE2 interaction and could be one of the reasons why this molecule is a successful inhibitor of this system.

The docking of AB-00011778 compound to the S-RDB protein (Figure 2B) in the absence of ACE2 led to a score of 89.12. The obtained docking pose shows the formation of a strong hydrogen bond between Gly502 and one of AB-0001778 ether groups, with a length of 1.90 Å. There is also the formation of π-π stacking interactions between Tyr505 the indole group and two of the ligand's phenyl groups. Finally, Lys417, Leu455, Asn501 and Tyr505 are involved in hydrophobic interactions with AB-00011778. Gly502 and Tyr505 have been implicated in MD studies of the S-RDB/ACE2 interface in very stable hydrogen bonds between with ACE2, with Gly502 interacting with Lys353 and Tyr505 with ACE2' Glu37, during 57 % and 56 % of simulation time, respectively ([2]). Hence, the interactions of AB-00011778 compound with these two residues are likely significantly interfere with the S-RDB/ACE2 association.

Additionally, the ability of AB-00011778 compound in binding to ACE2 competitively with the spike protein was evaluated. Both molecules were docked against the ACE2 receptor, defining as potential binding region the surface of the ACE2 that is known to interact and recognize the spike protein. Results are presented in Figure 4, showing that AB-00011778 compound can bind ACE2 with strong binding scores that are comparable to those observed when docked to S-RDB/ACE2 interface and to S-RDB alone.

The docking of AB-00011778 compound to ACE2 alone resulted in a score of 81.41 (GOLD/PLP). The most stable pose reveals the formation of multiple hydrogen bonds between ACE2 and AB-00011778 compound: (1) between Tyr41 and the nitrogen from the central pyridine group, with a length of 1.66 Å; (2) between Gln325 and Gly326 interacting with the oxygen from one of the ligand's ether groups, with a length of 2.04 Å and 3.09 Å respectively ; (3) between Asn330 and one oxygen from one of the sulfonyl groups, with a distance of 2.10 Å. One can also observe the formation of one π-cation interaction between Lys353 and phenyl ring from one of the indole groups. Glu37, Tyr41, Thr324, Gln325 and Lys353 are involved in hydrophobic interactions. Glu37, Tyr41 and Lys335 have also been previously implicated (Lapaillerie et al. ([2])) in important hydrogen bonds to the Spike protein, also suggesting that the interaction of AB-00011778 with these residues could interfere with the ACE2 ability to bind the Spike protein.

### Effect on the S-RBD/ACE2 association

To confirm that the selected drugs may physically prevent the formation of the S/ACE2 complex we next performed an *in vitro* alphaLISA assay using the recombinant S minimal binding domain (RBD) fused to a 6x-His tag (S-RBD(His)₆) and human ACE2 protein (ACE2-Biot).

For this, the AlphaLISA assay previously set up ([2]) was used and the binding conditions were adapted for use in 384-well microplate in a final reaction volume of 20 µl. The optimal concentration of 3 nM for each recombinant protein was previously established with cross-titration experiments. For the competition assay, increasing concentrations (1 to 25µM) of AB-00011778 drug was preincubated with S-RBD(His)₆ in the plate before being mixed with ACE2-Biot for 2h. Anti-6XHis acceptor and streptavidin donor beads were used for complex capture after 2 h of incubation of the two partners. As reported in Figure 5A, the AlphaLISA data confirmed that AB-00011778 compound could similarly interfere with the formation of the RBD/ACE2 complex reaching in both cases 50% inhibition at 25 µM of drugs. TruHlts counterassay experiment did not show any significant effect of the compound on the AS signal confirming the specific inhibition of the S-RBD/ACE2 interaction (data not shown). In order to get additional information about the effect of the drugs on the kinetic of the S-RBD/ACE2 interaction we performed biolayer interferometry (BLI) experiments as setup previously ([2]). In these competition binding assays, biotinylated ACE2 was loaded onto streptavidin biosensors and binding kinetics were carried out with 50 nM of S-RBD(His)₆ preincubated with increased concentrations of compound AB-00011778 as described in the Materials and Methods section. A strong binding of S-RBD(His)₆ to ACE2 was observed as indicated by the wavelength shift in the BLI sensorgrams (Figure 5B). The kinetic binding parameters (kₐ, k_{d}) and equilibrium dissociation constant (K_{D}) were determined after local fitting of the association and dissociation curves using a 1:1 binding model with ForteBio Blitz pro 1.1 software. The K_{D} was found to be 3.42 ±0.49 nM confirming previous data (([2]); Lan et al. (2020) Structure of the SARS-CoV-2 spike receptor-binding domain bound to the ACE2 receptor. Nature, 581, 215-220 ([3])). Addition of AB-00011778 compound inhibited the interaction leading to a strong drop of the K_{D} to 111 ±16 nM and 68.8 ± 5.1 nM respectively (see Figure 5C). Determination of the kinetic constants in the absence or in the presence of drugs showed that the compounds interfered on both the S-RBD/ACE2 association and dissociation steps, indicating that they can not only prevent the formation of the S-RBD/ACE2 complex and also decrease its stability, in agreement with the docking models.

In a cellular context both ACE2 and S are embedded in a membranous environment, i.e. the viral envelop and the cellular membrane. Thus, we next investigate whether the compounds could also prevent the S/ACE2 interaction in a more physiological context. For this purpose we used the cellular S-RBD/ACE2 interaction model developed previously ([2]). This model allows to monitor the association between recombinant S-RBD protein and the cellular membranous ACE2 protein at the HEK293T-ACE2 cells surface by anti-S immunofluorescence (Figure 6A). Flow cytometry analysis allows the quantification of the global S-RBD association to ACE2 protein (Figure 6B) and competition with soluble ACE2 protein confirms the specificity of the interaction as well as validates the assay for testing potential S/ACE2 inhibitors (Figure 6C). Addition of AB-00014778 compound decreased the efficiency of S-RBD/ACE2 association at the surface of the cell (Figure 6D). The AB-00014778 compound was found efficient, in agreement with data obtained in infectivity assays reported in Figure 1.

Altogether, the data obtained demonstrate that the selected drug blocks the early interaction between S and ACE2 supporting, in concert with the data obtained with pseudoviruses, their effect on the early entry pathway mediated by this interaction.

### Effect of on wild type SARS-CoV-2 infectivity on naturally susceptible human cells.

We next investigate whether this S/ACE2 inhibitory effect could be sufficient for blocking the replication of wild type circulating SARS-CoV-2 strains in different cell lines naturally susceptible to the infection.

The drug was tested in different pulmonary cell lines overexpressing, or not, the human ACE2 viral receptor and infected with the SARS-CoV-2 reference strain. As reported in Figure 7A, both molecules induced an inhibition of the viral replication in A549-ACE2 cells with an EC₅₀ for AB-00011778 compound of 2.5µM and 0.25µM, respectively. Drugs were then further tested in infection assays using the Calu3 human lung cancer cell lines that naturally express the ACE2 receptor. As reported in Figure 7B an inhibitory effect of the viral replication was confirmed for both drugs leading to a significant 50% inhibition of the viral replication at concentration around 1µM.

As reported in Table 1, based on the apparent IC₅₀ and CC₅₀ reported in the different cell lines the two compounds reached selectivity index (SI) higher than 50 making them good candidates and anti-SARS-coV-2 selective drugs. AB-00011778 was found a promising molecules reaching a SI>200 in A549-ACE2 cells. We, thus, further studied the molecular mechanism underlying the viral inhibition by the selected drug.

**Table 1. IC₅₀, CC₅₀ and SI of AB-00011778 compound. The CC₅₀ was calculated using four-parameter variable slope sigmoidal dose-response models based on MTT assays.**

| | 293-ACE2 | A 549-ACE2 | Calu3 |
|---|---|---|---|
| IC₅₀ | 1 | 0.25 | 1 |
| CC₅₀ | >50 | >50 | >50 |
| SI | >50 | >200 | >50 |

### Effect of the selected drugs on SARS-CoV-2 circulating variants forms.

To determine whether the selected drug could be effective on the current major SARS-CoV-2 variants we first performed an *in silico* modelling and molecular dynamics simulations of the SARS-CoV-2 S/ACE2 complex variants alpha, beta, gamma and delta.

Models for the S-RDB/ACE2 complex formed with the different SARS-CoV-2 mutants were prepared starting from the X-ray structure 6M0J (resolution 2.45 Å) ([3]), which represents the original SARS-CoV-2 spike protein receptor binding domain complexed with ACE2. Mutant variants were prepared by modelling the interface mutations using the mutagenesis feature in Pymol 1.7.2.1 software, using the Dunbrack rotamer libraries available (Shapovalov and Dunbrack (2011) A smoothed backbone-dependent rotamer library for proteins derived from adaptive kernel density estimates and regressions. Struct. Lond. Engl. 1993, 19, 844-858 ([5])). Selection of the initial amino-acid conformation of each mutant was based on the dominant conformer predicted, excluding clashes with other residues. This computational mutagenesis protocol has been previously used with success in the study of other enzymes (Serrano et al. (2021) Compensatory epistasis explored by molecular dynamics simulations. Hum. Genet., 140, 1329-1342 ([6]); Improving the Catalytic Power of the DszD Enzyme for the Biodesulfurization of Crude Oil and Derivatives - Ferreira - 2017 - Chemistry - A European Journal - Wiley Online Library ([7])). The mutations modelled are described in Table 2.

**Table 2. Description of the SARS-CoV-2 mutants modelled in the evaluation of the S-RDB/ACE2 interaction**

| Mutant | Common Name | Pango Name | Mutations in the S-RDB |
|---|---|---|---|
| Alpha | UK | B.1.1.7 | N501Y |
| Beta | South Africa | B.1.351 | K417N, E484K, N501Y |
| Gamma | Brazil | B.1.1.28.1 | K417T, E484K, N501Y |
| Delta | India | B.1.617.2 | L452R, T478K |

All the 4 systems were subject to the same molecular mechanics minimization and molecular dynamics procedure previously described, employing AMBER and the ff14sb force field. Molecular dynamics simulations were run for 400 ns for each mutant. Final trajectories were analyzed in terms of backbone root-mean-square deviation (RMSD), hydrogen bonds formed and cluster analysis. From the cluster analysis performed on each mutant, a representative structure from the dominant cluster of conformations obtained for each mutant was selected using cpptraj from AmberTools and prepared for the molecular docking stage.

AB-00011778 compound was docked into the structures of each of the mutants using the PLP scoring function from GOLD with 500 independent genetic algorithm runs. Molecules were docked into: (1) the S-RDB/ACE2 interface; (2) S-RDB alone. Table 3 presents the best scores obtained when docking the two molecules to the different variants, compared with the values obtained against the initial non-mutated S-RDB/ACE2.

**Table 3. GOLD/PLP docking scores of AB-00011778 compound to the structures of the different SARS-CoV-2 mutants**

| **Molecules** | **AB-00011778** | |
|---|---|---|
| **Targets** | **S-RDB/ACE2** | **S-RDB** |
| Initial | 92.05 | 89.12 |
| Alpha | 89.75 | 92.99 |
| Beta | 78.14 | 92.67 |
| Gamma | 91.97 | 93.96 |
| Delta | 81.57 | 87.30 |
| **Average** | **86.7 ± 5.7** | **91.2 ± 2.6** |

The results presented in Table 3 show that both molecules are able to bind with high docking scores and consistently to the different mutants, reinforcing the idea that these molecules can be equally effective in impairing the S/ACE2 association in the different mutants.

To confirm this we tested AB-00011778 drug on the three major circulating forms of SARS-CoV2 namely the original Wuhan strain and the Alpha and Delta viruses in pulmonary A549-ACE2. The molecule was found to be also able to block the viral replication of both viruses with very similar efficiency leading IC₅₀ = 0.5-1µM. Noticeably, as shown in Figure 8, similar effects were observed in A549 cells expressing both ACE2 and the TMPRS2 membrane protease suggesting that the drug is efficient in inhibiting the natural SARS-CoV-2 entry pathway as expected from its effect on the early S/ACE2 association.

### Selection of efficient AB00011778 analogs

Based on the chemical structure of the molecule we design several derivatives from the initial AB-00011778 compounds (see their names and structures in Figure 9).

The selected compounds were first screened in the SARS-CoV-2 LVs infectivity assay as in Figure 10 using 10µM concentration. Among all the tested drugs three were found cytotoxic and could not be studied further. Two derivatives, namely AB-0001178_2 and AB-0001178_6 were more efficient in inhibiting the LV infectivity in HEK-293T-ACE2 cell model than the leading compound (Figure 10A). Infectivity assay performed with wider concentration ranges confirmed that both analogs were 4-6 times more efficient that the initial molecule (Figure 10B).

### Analysis of the Interactions of the Analogs with S-RDB/ACE2, S-RDB alone and comparative docking to ACE2 alone.

One of the hypotheses emerging from the experimental studies was the ability of the selected molecules in binding simultaneously, targeting different components of the interface, namely (1) the S-RDB/ACE2 interface; (2) S-RDB alone; (3) ACE2 alone. It has been suggested that the combined effect of these three association modes could contribute for the inhibitory effect observed. To evaluate that hypothesis AB-00011778 compound, were docked into: (1) the S-RDB/ACE2 interface; (2) S-RDB alone; (3) ACE2 alone. For (2) and (3) the cavity for docking was defined based on the known binding region of the other partner in the complex formed (i.e. of ACE2 for the S-RDB model (2) and S-RDB for the ACE2 model (3), plus a radius of 10 Å. The PLP scoring function from GOLD was used with 500 independent genetic algorithm runs. The same procedure was later extended to AB-00011778_2 and AB-00011778_6. Results are presented in Table 4.

**Table 4. GOLD/PLP docking scores of AB-00011778, AB-00011778_2 and AB-00011778_6 to the different possible targets involved in the S-RDB/ACE2 association.**

| Molecules | S-RDB/ACE2 | S-RDB | ACE2 |
|---|---|---|---|
| AB-00011778 | 92.05 | 89.12 | 81.41 |
| AB-00011778 _ 2 | 72.15 | 63.90 | 61.50 |
| AB-00011778 _ 6 | 69.56 | 60.34 | 59.74 |

The results presented in Table 4 show that although the best docking scores are observed for all molecules against the S-RDB/ACE2 interface, all these molecules exhibit high affinity towards S-RDB alone and interestingly towards ACE2 alone. For AB-00011778_2 and AB-00011778_6, the scoring values obtained for S-RDB and ACE2 alone are very similar. Globally, these results agree with the proposed hypothesis that these molecules can target the different components involved in the S-RDB/ACE2 association, suggesting that the observed effect can results from the combined action of different binding modes to the interface and to the individual components.

In terms of interaction analysis (see Figure 11-12), the best docked pose of AB-00011778_2 in the interface between S-RDB/ACE2 yielded a score of 72.15 using the GOLD/PLP scoring function. Multiple hydrogen bonds are established. Residues ACE2 Lys353 and S-RDB Ser494 and Gly496 interact with one of the phenol groups, with interaction distances of 1.84 Å, 3.28 Å and 2.67 Å. ACE2 Asn33 interacts with one of the tertiary amines with a distance of 2.38 Å. Lastly, S-RDB Arg403 interacts with the other tertiary amine with distance of 3.60 Å. Residues ACE Asp30 and S-RDB Glu406 also interact the tertiary amines. ACE2 His34 establishes π-π stacking interactions with the indole group and S-RDB Lys417 interacts with the other indole group via π-cation interactions. Finally, ACE2 Glu37 and S-RDB Lys417 perform hydrophobic interactions.

The docking of AB-00011778_2 to S-RDB alone yielded a score of 63.90. Hydrogen bonds are formed between Gly496 and one of the phenol groups, with a length of 1.82 Å. The other phenol group established hydrogen bonds with Gln409 and Lys417 with a length of 2.55 Å and 2.20 Å. Tyr505 interacts with the indole group via π-π stacking interactions. Hydrophobic interactions occur between AB-00011778_2 and Lys417.

The best docked pose of AB-00011778_6 to the S-RDB/ACE2 interface generated a score of 69.56. One can observe multiple hydrogen bonds being formed between AB-00011778_6 and the two proteins. ACE2 Asp38 and S-RDB Gly496 establish hydrogen bonds with one of the phenol groups, with an interaction distances of 4.02 Å and 2.09 Å respectively. ACE2 Asn33 interacts with the tertiary amine group with a distance of 2.57 Å. Finally, S-RDB Arg403 forms a 2.52 Å long hydrogen bond with the nitrogen from the indole group. One salt bridge is formed between ACE2 Asp30 and the tertiary amine and a π-cation interaction occurs between Lys417 and one of the indole groups. ACE2 Glu37 and Tyr78 also interact with A03-6 via hydrophobic interactions.

AB-00011778_6 docked with S-RDB had a score of 60.34. The interactions of AB-00011778_6 with S-RDB are very similar to what was observed with AB-00011778_2. Gly496 interacts with one of the phenol groups, with a length of 1.84 Å, and Gln409 and Lys417 interact with the other phenol group, with interaction lengths of 2.58 Å and 2.14 Å. Tyr505 also interacts with one of the indole groups via π-π stacking interactions. Lys417 interacts with A03-6 via hydrophobic interactions.

Next, the interaction of the two analogs with the ACE protein alone was examined (see Figure 13-14). The best conformation of the docking of AB-00011778_2 to the ACE2 protein alone resulted in a score of 61.50. Hydrogen bonds are formed between Lys31 and one of the phenol groups, with a length of 2.69 Å, and between Tyr83 and the nitrogen from the central pyridine group, with a distance of 2.84 Å. Asp30 forms a salt bridge with one of the tertiary amines and Lys31 establishes π-cation interaction with one of the indole groups. Hydrophobic interactions occur between AB-00011778_2 and ACE2 Gln24 and Lys31.

The docking of AB-00011778_6 to ACE2 resulted in a best score of 59.74. A hydrogen bond is formed between Asp30 and one of the phenol groups, with a distance of 1.64 Å. Other hydrogen bond established between Lys31 and the central pyridine group, with a length of 1.96 Å. Glu35 interacts with the nitrogen from one of the indole groups, with a distance of 3.08 Å. One last hydrogen bond, with a length of 1.84 Å is formed between Lys353 and the other phenol group. Finally, hydrophobic interactions happen between AB-00011778_6 and Thr27, Lys31, His34 and Asp38. Three residues are of major importance since they are reported to be involved in the interaction between ACE2 and S-RDB. These are Glu35, Asp38 and Lys353.

Taken together all these data indicate that the selected drugs are efficient on all the current SARS-CoV-2 strains and show an original entry inhibition mechanism by possibly acting simultaneously on distinct S/ACE2 interfaces.

### Discussion

The SARS-CoV-2 S/ACE2 mediated entry remains a major antiviral target not yet exploited in therapy. We identified drugs showing potent entry inhibition in lentiviral-based pseudovirus infectivity assays. The specific inhibition of SARS-CoV-2 LV infectivity in comparison with VSVg LVs suggested that the selected drugs target the early S/ACE2 dependent entry process recapitulated in this model. The simplest explanation for the inhibition mechanism is a block in the S/ACE2 association due to binding of the drug on the S/ACE2 interface. Biochemical approaches confirmed a direct effect of the drugs in blocking the S-RBD/ACE2 interaction. The two compounds were also shown to inhibit the interaction between the soluble S-RBD and the ACE2 receptor expressed at the surface of HEK-293T-ACE2 cells confirming their capability to interfere with the formation of the S/ACE2 complex in a physiological condition. Detailed analysis of the effect of the drugs on the kinetic parameters of the S/ACE2 complex formation indicated that they could both prevent the association between the two partners but also decrease the stability of the complex. These data are in full agreement with the molecular docking calculation performed on the S/ACE2 complex structure and on the S-RDB and ACE2 proteins alone indicating that both drugs could make contact with the viral and cellular proteins.

In order to determine whether the selected drug could be potential antiviral lead compounds we tested them on the wild type viral replication using different cell lines including naturally susceptible cells. Data confirm that both drugs were able to block or reduce the replication of the virus in pulmonary A549-ACE2 and Calu3 cells. AB-00011778 compound kept an efficient blocking effect reaching 60-40% inhibition at 1µM. Overall, both drugs showed IC₅₀ between 0.25 to 5µM among all the cell lines without showing significant cyto-toxicity allowing them to reach selectivity indexes >10. Notably, the AB-00011778 compound showed an inhibitory effects in all the cell lines with SI>50-200. Further analysis of the inhibitory effect of the AB-00011778 compound showed that it could also block the replication of the main major circulating forms Wuhan, Alpha and Delta of SARS-CoV-2 in pulmonary cells including those expressing both ACE2 and TMPRS2 showed to dictate the viral entry (Koch et al. (2021) TMPRSS2 expression dictates the entry route used by SARS-CoV-2 to infect host cells. EMBO J., 40 ([8])). These data indicate that the drug is able to block the different entry routes of the virus. Taken together these data pointed out the AB-00011778 compound as a good lead compound candidate for further developments.

Deeper molecular docking analyses allowed us to design new potential effective molecules. Especially, the indole structure of AB-00011778 provides interesting framework for the design of novel compounds using previously reported synthesis approaches ([1]). Based on these new designs we selected two additional derivatives, AB-00011778_2 and AB-00011778_6 four to six times more efficient than the initial compounds in blocking SARS-coV-2 LV infectivity in HEK293T-ACE2 cells. This fully confirmed the lead compound position of AB-00011778 and the possibility to identify new molecules with higher inhibitory capability during structure-activity relationship studies. Noticeably, the selection of molecules active on wild type virus replication also validate the overall strategy and screening pipeline used in this work. This suggest that the list of selected compounds may provide additional potential candidates as SARS-CoV-2 entry inhibitors that remain to be validated.

Interestingly, the molecular modelization studies reported in this work suggest that the selected drugs may interact both with S and ACE2 protein. This fully agree with the BLI data indicating that the molecules could interfere the formation of the S/ACE2 complex as well as decreasing the stability of the formed complex. This may constitute an original inhibition mechanism of simultaneous blocking of various S/ACE2 interfaces increasing the inhibition potency in addition to decreasing the resistance emergency.

Apart, from the recently licensed remdesivir no effective drugs has been validated as potential COVID treatment. The compounds reported here constitute the first molecules acting on the SARS-CoV-2 entry step by targeting specifically the S/ACE2 interfaces making them very promising agent. The lack of cytotoxicity observed for these molecules in various cell lines also open the way for future animal model studies and clinical trials in order to fully evaluate their antiviral property and therapeutic potency. Further characterization of the molecular mechanism underlying the inhibition of S/ACE2 interaction by the selected drugs will certainly allow the optimization of this original antiviral process and lead to new promising agents.

### MATERIALS and METHODS

### Molecular Docking of the LEAD compounds

For the molecular docking of AB-00011778 compound, the PLP scoring function from GOLD was used with 500 independent genetic algorithm runs. Molecules were docked into: (1) the S-RDB/ACE2 interface; (2) S-RDB alone; (3) ACE-2 alone. For (2) and (3) the cavity for docking was defined based on the known binding region of the other partner in the complex formed (i.e. of ACE2 for the S-RDB model (2) and S-RDB for the ACE2 model (3)), plus a radius of 10 Å. The same procedure was later extended to AB-00011778_2 and AB-00011778_6. Analysis of the interactions was done with the help of Protein-Ligand Interaction Profiler (Adasme et al. (2021) PLIP 2021: expanding the scope of the protein-ligand interaction profiler to DNA and RNA. Nucleic Acids Res., 49, W530-W534 ([9])).

### Chemicals

Bis-indolylpyridines and their derivatives have been synthetized as previously reported by Jacquemard et al. ([1]) and Mahboobi et al. ([13]).

### Proteins and antibodies

SARS-CoV-2 (438-516) S-RBD(HiS)₆ including the minimal receptor binding motif previously described (([3]); Wrapp et al. (2020) Cryo-EM structure of the 2019-nCoV spike in the prefusion conformation. Science, 367, 1260-1263 ([10])) and biotynilated human ACE2 have been purchased from Fisher Scientific (respective references 16534204 and 16545164). Monoclonal anti-6X His tag antibody has been purchased from ABCAM (reference ab18184, dilution 1/200). Anti α-tubulin antibody has been purchased from SIGMA (reference T6199, dilution 1/500). Secondary goat anti-mouse antibody coupled to AlexaFluor 488 has been purchase from Fisher Scientific (Reference Allo2g, dilution 1/400).

### AlphaLISA binding assays

The AlphaLISA assay development was performed as previously described ([2]) using the recombinant S minimal binding domain (RBD) fused to a 6x-His tag (S-RBD(His)₆) and biotinylated human ACE2 protein (ACE2-Biot). The binding conditions were adapted for use in 384-well plate (white OptiPlate, reference 6007290, PerkinElmer, Waltham, MA) in a final reaction volume of 20 µl. An optimal final concentration of 3nM for each recombinant protein was previously established with cross-titration experiments. Proteins were diluted in the binding buffer (phosphate-buffered saline (PBS) pH 7.4, 0.1% (w/v) bovine serum albumin). For the AlphaLISA competition assay, increasing concentrations of AB-00011778 drug (1 to 25 µM, 1% DMSO final concentration) were preincubated with S-RBD(His)₆ for 30 min before being mixed with ACE2-Biot for 2h with rotation at room temperature. Next, 5µL of anti-6X His acceptor beads (Perkin Elmer, reference AL178) were added and after 1h of incubation at RT with rotation, 5 µL of streptavidin donor acceptor beads (Perkin Elmer, reference 6760002) were also mixed to the wells. This established a final concentration of 20 µg/mL for each bead. The plate was then incubated for 1h at RT in the dark before the AlphaLISA signal was detected using an EnSpire Multimode Plate Reader (Perkin Elmer). Negative control with binding buffer was used to control the assay quality. Data were analyzed with GraphPad Prism 5.01 software. The AlphaLISA binding data obtained with compounds were compared to the 1% DMSO control condition.

For hits validation, TruHits counterassay kit (reference AL900D, PerkinElmer) was performed by incubating AB-00011778 drug with biotin-BSA acceptor and streptavidin donor beads with the same working dilutions than in the AlphaLISA binding assay. In this validation assay, if the compound causes a decrease of the signal, it means that it interferes with AlphaLISA readout and therefore it is not relevant to the specific target of interest.

### Bio-Layer Interferometry binding assays.

Bio-Layer Interferometry (BLI) experiments were performed on a BLItz instrument (Sartorius, Göttingen, Germany) to measure the binding of S-RBD(His)₆ to ACE2-Biot. BLI assay was set up as previously described ([2]) with some adaptations for the competition binding analysis with drugs. All sample dilutions and baseline steps were carried out using the same reaction buffer (phosphate-buffered saline pH 7.4, 0.1% (w/v) bovine serum albumin, 1% (v/v) DMSO). Streptavidin biosensors (Sartorius, reference 18-5019) were first pre-wet for 10 min with the reaction buffer and 1 µM of ACE2-Biot was then loaded onto the coated biosensors for 500 s in order to reach a binding value of ~ 3 nm. For the competition kinetic analyses, protein samples were prepared at the concentration of 50 nM S-RBD(His)₆ in accordance with our previous BLI binding studies and were preincubated for 15 min at room temperature with increased concentrations (0-25µM) of AB-00011778 drug. Binding kinetics were divided in three steps. Firstly, baseline with the reaction buffer was measured for 60 s. For the association step, each loaded biosensor was dipped into into S-RBD sample with drugs or DMSO as control for 300 s with a 2200 rpm shaking speed. Finally, the dissociation step of the bound S-RBD(His)₆ was monitored by dipping the biosensor back into the reaction buffer for 300 s. Systematic baseline drift correction was done by subtracting the shift recorded for biosensor loaded with ACE2 but incubated with reaction buffer. The association and dissociation experimental curves were local fitted using a 1:1 binding model with the Blitz pro 1.1 software and the kinetic binding parameters (kₒₙ, k_{off}) were determined as means of two to three independent experiments. The equilibrium dissociation constant (K_{D}) calculated as K_{D} = k_{off}/kₒₙ. Sensorgrams curves were plotted using Prism 5.0 software (Graphpad Software, La Jolla, CA).

### Cells and lentiviral vectors production

Lentivirus vector production was done by the service platform Vect'UB, (INSERM US 005 - CNRS UMS 3427- TBM-Core, Université de Bordeaux, France). Lentiviral particles were produced by transient transfection of HEK293T (human embryonic kidney cells according to standard protocols. In brief, subconfluent HEK293T cells were cotransfected with lentiviral genome (psPAX2) (gift from Didier Trono (Addgene plasmid # 12260 ), with an envelope coding plasmid (pMD2G-VSVG or wild type SARS-CoV-2 Spike protein (HDM_IDTSpike_fixK (kind gift from Bloom's laboratory, ([4]) and with vector constructs (44 :pRRLSIN-PPT-hPGK-eGFP-WPRE or pHAGE_EF1aInt_ACE2_WT) by calcium phosphate precipitation. LVs were harvested 48h posttransfection and concentrated by ultracentrifugation. Viral titers of VSV-g pseudotype pLV lentivectors were determined by transducing HEK293T cells with serial dilutions of viral supernatant and eGFP expression was quantified 5 days later by flow cytometry analysis. For SARS-CoV-2 Spike pseudotype, p24 antigen levels were measured in the concentrated viral supernatants by an enzyme-linked immunosorbent assay (Innotest HIV Ag nAb; Fugibio, France) and viral titers were estimated by comparing p24 antigen levels of each lentiviral supernatant with a similar VSV-g pseudotype lentiviral supernatant produced simultaneously.

HEK293T-ACE2 cell lines have been generated by lentiviral transduction using pHAGE_EF1aInt_ACE2_WT plasmid (kind gift from Bloom's laboratory ([4]). HEK293T cells (200 000 cells) were then transduced with optimized concentration of ACE2 lentiviral particles in 6-well plates. The efficacy of transduction was assessed using real time PCR ten days post infection. ACE2 provirus DNA from this cell line was quantified by q-PCR using the ΔCt method as compared with 1 copy cell line. DNA from two different cell clones (human 293T and K562 cells), containing a single integrated copy of the provirus, was used as a normalized cell line. HEK293T were cultured in DMEM medium supplemented with 1% penicillin-streptomycin and FBS (Fetal Bovine Serum) 10%.

A549 cells (Human alveolar basal epithelial carcinoma), Calu3 (human bronchial epithelial carcinoma) and Vero E6 cells (African green monkey kidney cells) were maintained in Dulbecco Modified Eagle Medium (DMEM; Invitrogen Life Technologies) supplemented with 10% heat-inactivated fetal bovine serum (FBS) and 1% penicillin/streptomycin (P/S) and 1% GlutaMAX (Life Technologies).

In cellulo imaging of the SARS-CoV-2 S-RBD/ACE2 interaction.

10 000 HEK293T and HEK293T-ACE2 plated on glass coverslips pretreated with poly-L-Lysine solution 0.01% (SIGMA ref P4832) 5 minutes at room temperature. After 24 hours the RBD recombinant protein (4-40 pmoles) was added to the cells in 100µl PBS. Cells were then washed and fixed (PFA 4%) at different time points. Cell imaging was performed as described above.

For quantification of the S-RBD/ACE2 interaction in cellular context 45 000 HEK293T and HEK293T-ACE2 cells were incubated 45 minutes at 37°C in 100 µl Dulbecco's Modified Eagle's Medium (DMEM) and increasing concentrations of RBD recombinant protein. One ml of PBS was added the cells were centrifugated 5 minutes at 2 500 rpm. 50 µl of anti-His antibody 1/200 in DMEM was added and the cells were further incubated 45 minutes at 37°C. After PBS washes 50 µl of a 1/400 DMEM solution of secondary antibody was added and cells were incubated 30 minutes at 37°C. After PBS washes the cells were resuspended in 200µl PBS, FBS 2% EDTA 2mM and the percentage of FITC positive cells was quantified by flow cytometry.

### Cytotoxicity assay

MTT cell viability assay was performed following furnisher protocols. Cells were seeded at the density of 20,000 cells/well in a 96 well plate containing 100 µL complete DMEM (Gibco, USA) supplemented with 10% FBS (Gibco, USA) and 1% Penstrep (Gibco, USA). Cells were incubated for 12 hours at 37° C in humidified 5% CO₂ incubator for adherence. After 12-hour incubation, the media was replaced with fresh media, and cells were treated with compounds. Untreated cells were considered as negative control, DMSO treated cells were considered as the vehicles. After the treatment, cells were incubated at 37 ° C in humidified 5% CO₂ incubator. 48-hour post-treatment, 20 µL of MTT substrate (5 mg/mL) was added in each well and incubated for 4 additional hours at 37° C in the dark. The media was then carefully removed, and 492nm absorbance was measured.

### SARS-CoV2 production

SARS-CoV-2 strain 220_95 (EPI_ISL_469284) was isolated from nasopharyngeal swab specimens collected at Service de Virologie (Hospital Saint Louis, Paris) and grown as previously described (Onodi et al. (2021) SARS-CoV-2 induces human plasmacytoid predendritic cell diversification via UNC93B and IRAK4. J. Exp. Med., 218 ([11])). Briefly, virus was propagated on Vero E6 in DMEM-2% (DMEM supplemented with 2% FBS, 1% P/S, 1% GlutaMAX, and 25 mM Hepes). viruses were purified through a 20% sucrose cushion by ultracentrifugation at 80,000xg for 2 hours at 4°C. Pellets were resuspended in HNE 1X (HEPES 25mM, NaCl 100mM EDTA 0.5mM) aliquoted and stored at -80°C.

### SARS-CoV-2 infection assay

A549-Ace2 and Calu3 cells grown in 12-well plates were challenged with SARS-CoV-2 at a MOI of 0.05 in the presence of the indicated drug or with DMSO as a control. After 3 hours, cells were washed once with PBS and incubated with fresh media. Compounds were maintained throughout the course of infection.

To assess productive viral replication, we quantified the infectious viral particles release during infection 24 hours post-infection by RT-qPCR. Viruses were first inactivated by incubating the supernatants v/v with 1% Triton X-100 (Sigma) in PBS for 30 min under agitation at RT. Yields of viral RNA were quantified by real-time qPCR by using SARS-CoV-2 specific primers targeting the E gene with the Luna^{®} Universal One-Step RT-qPCR Kit (New England Biolabs) in a LightCycler 480 thermocycler (Roche) according to the manufacturer's protocol. The number of viral genomes is expressed as PFU equivalent/ml and was calculated by performing a standard curve with a similarly treated supernatant from a viral stock with a known titer as described by Gordon et al (Gordon et al. (2020) Comparative host-coronavirus protein interaction networks reveal pan-viral disease mechanisms. Science, 10.1126/science.abe9403 ([12])).

### Reference List

1. Jacquemard,U., Dias,N., Lansiaux,A., Bailly,C., Logé,C., Robert,J.-M., Lozach,O., Meijer,L., Mérour,J.-Y. and Routier,S. (2008) Synthesis of 3,5-bis(2-indolyl)pyridine and 3-[(2-indolyl)-5-phenyl]pyridine derivatives as CDK inhibitors and cytotoxic agents. Bioorg. Med. Chem., 16, 4932-4953.
2. Lapaillerie,D., Charlier,C., Fernandes,H.S., Sousa,S.F., Lesbats,P., Weigel,P., Favereaux,A., Guyonnet-Duperat,V. and Parissi,V. (2021) In Silico, In Vitro and In Cellulo Models for Monitoring SARS-CoV-2 Spike/Human ACE2 Complex, Viral Entry and Cell Fusion. Viruses, 13.
3. Lan,J., Ge,J., Yu,J., Shan,S., Zhou,H., Fan,S., Zhang,Q., Shi,X., Wang,Q., Zhang,L., et al. (2020) Structure of the SARS-CoV-2 spike receptor-binding domain bound to the ACE2 receptor. Nature, 581, 215-220.
4. Crawford,K.H.D., Eguia,R., Dingens,A.S., Loes,A.N., Malone,K.D., Wolf,C.R., Chu,H.Y., Tortorici,M.A., Veesler,D., Murphy,M., et al. (2020) Protocol and Reagents for Pseudotyping Lentiviral Particles with SARS-CoV-2 Spike Protein for Neutralization Assays. Viruses, 12.
5. Shapovalov,M.V. and Dunbrack,R.L. (2011) A smoothed backbone-dependent rotamer library for proteins derived from adaptive kernel density estimates and regressions. Struct. Lond. Engl. 1993, 19, 844-858.
6. Serrano,C., Teixeira,C.S.S., Cooper,D.N., Carneiro,J., Lopes-Marques,M., Stenson,P.D., Amorim,A., Prata,M.J., Sousa,S.F. and Azevedo,L. (2021) Compensatory epistasis explored by molecular dynamics simulations. Hum. Genet., 140, 1329-1342.
7. Improving the Catalytic Power of the DszD Enzyme for the Biodesulfurization of Crude Oil and Derivatives - Ferreira - 2017 - Chemistry - A European Journal - Wiley Online Library.
8. Koch,J., Uckeley,Z.M., Doldan,P., Stanifer,M., Boulant,S. and Lozach,P. (2021) TMPRSS2 expression dictates the entry route used by SARS-CoV-2 to infect host cells. EMBO J., 40.
9. Adasme,M.F., Linnemann,K.L., Bolz,S.N., Kaiser,F., Salentin,S., Haupt,V.J. and Schroeder,M. (2021) PLIP 2021: expanding the scope of the protein-ligand interaction profiler to DNA and RNA. Nucleic Acids Res., 49, W530-W534.
10. Wrapp,D., Wang,N., Corbett,K.S., Goldsmith,J.A., Hsieh,C.-L., Abiona,O., Graham,B.S. and McLellan,J.S. (2020) Cryo-EM structure of the 2019-nCoV spike in the prefusion conformation. Science, 367, 1260-1263.
11. Onodi,F., Bonnet-Madin,L., Meertens,L., Karpf,L., Poirot,J., Zhang,S.-Y., Picard,C., Puel,A., Jouanguy,E., Zhang,Q., et al. (2021) SARS-CoV-2 induces human plasmacytoid predendritic cell diversification via UNC93B and IRAK4. J. Exp. Med., 218.
12. Gordon,D.E., Hiatt,J., Bouhaddou,M., Rezelj,V.V., Ulferts,S., Braberg,H., Jureka,A.S., Obernier,K., Guo,J.Z., Batra,J., et al. (2020) Comparative host-coronavirus protein interaction networks reveal pan-viral disease mechanisms. Science, 10.1126/science.abe9403.
13. Mahboobi et al. (Bis(1H-2-indolyl)methanones as a novel class of inhibitors of the platelet-derived growth factor receptor kinase; J. Med. Chem 2002 45 1002-1018.

## Claims

1. A compound comprising a compound having formula (I) below: 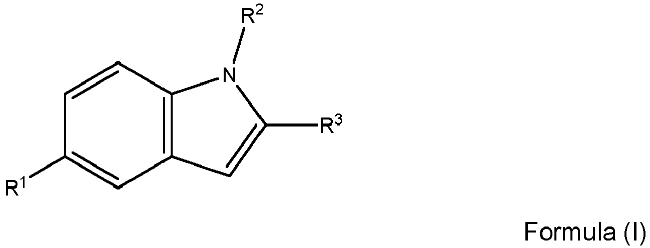 wherein:
- R¹ is chosen from the group comprising: 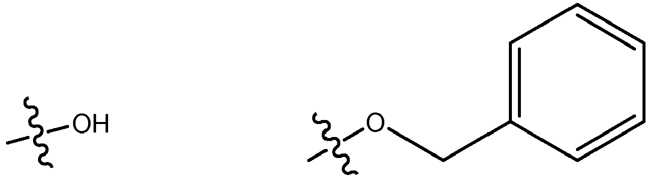 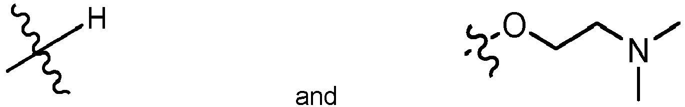
- R² is chosen from the group comprising: 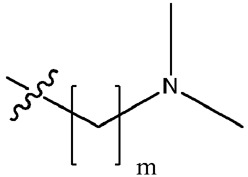 m being an integer from 2 to 6, 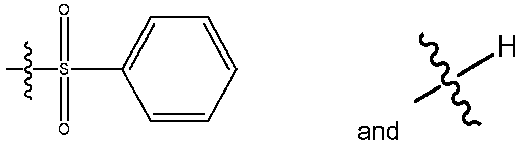
- R³ is chosen from the group comprising: 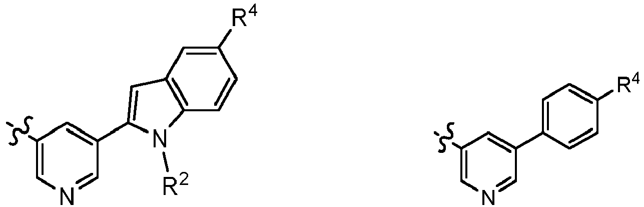 and 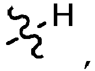 in which R² is defined as above,
and R⁴ is chosen among : 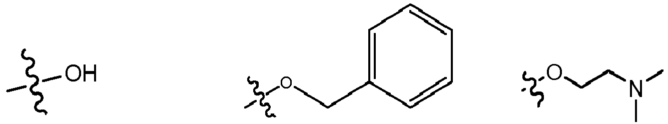 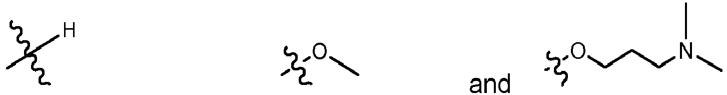 or a pharmaceutically acceptable salt thereof,
for use in the prevention or in the treatment of an infection.

2. The compound according to claim 1, wherein:
- R² is chosen from the group comprising: 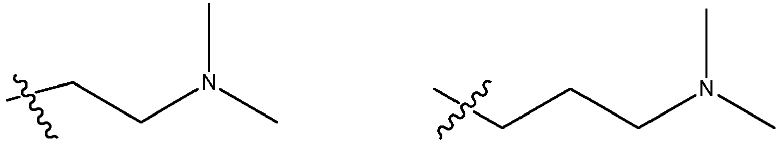 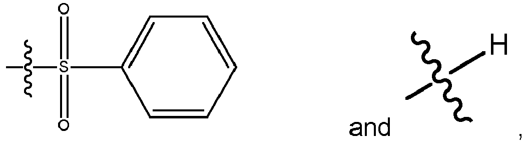
- R³ is chosen from the group comprising:

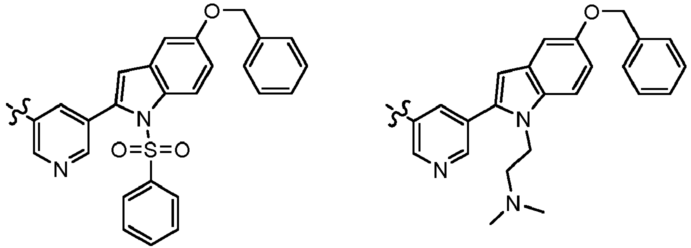

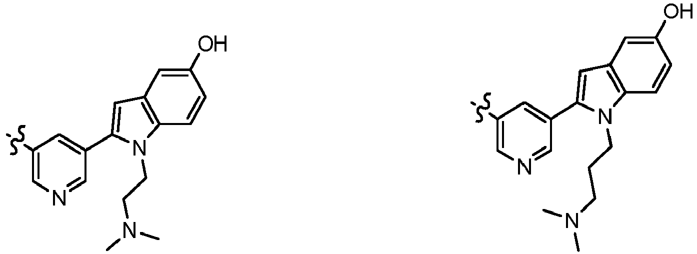

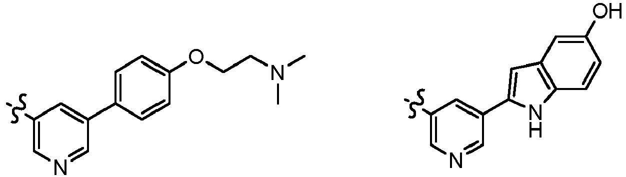

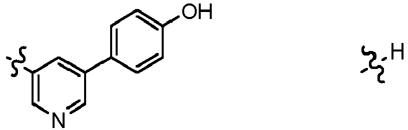

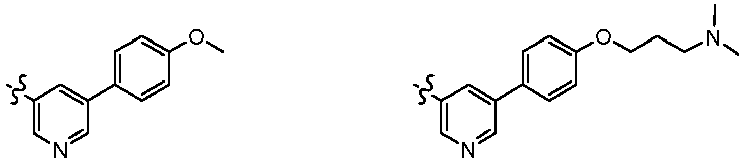

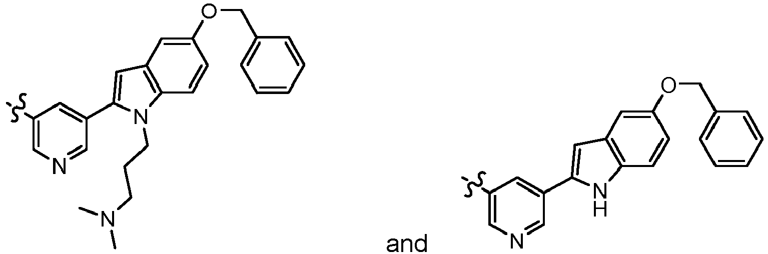

3. The compound according to claim 1 or 2, which is chosen among: 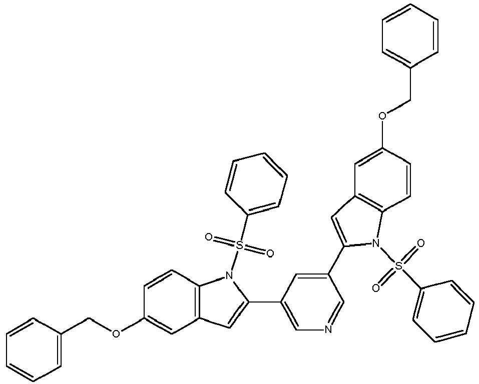 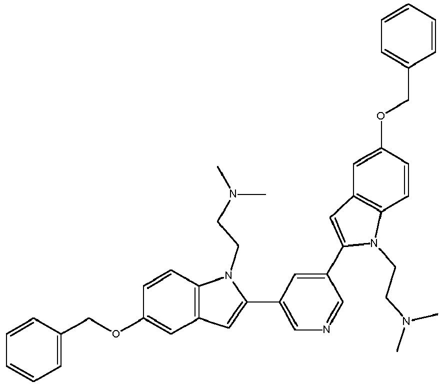 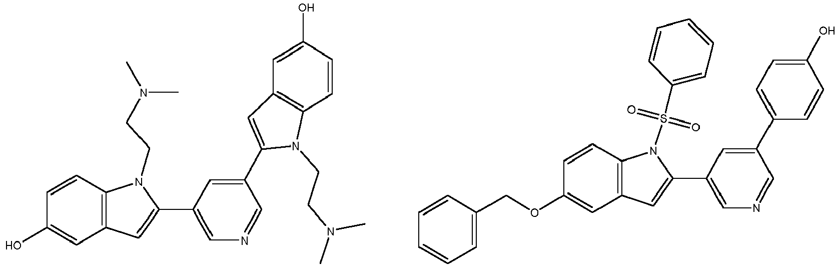 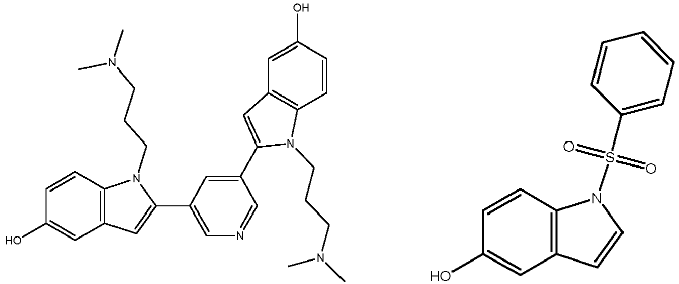 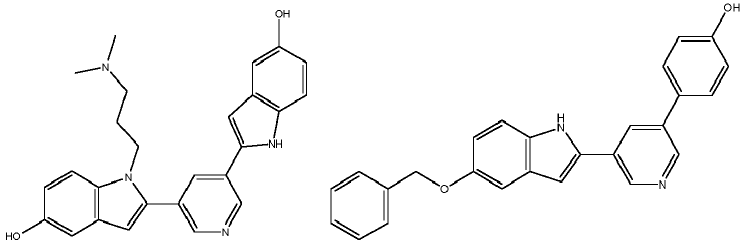 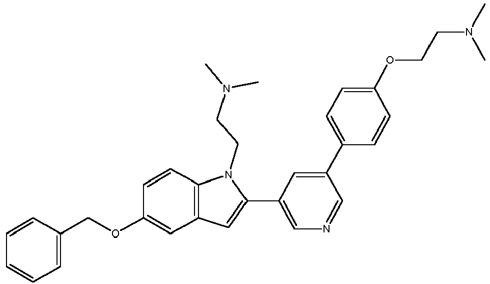 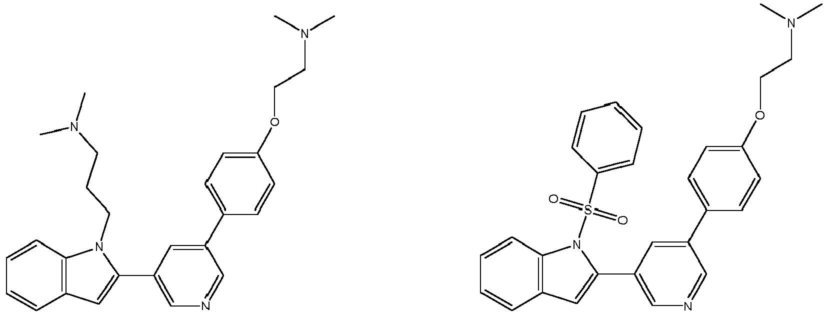 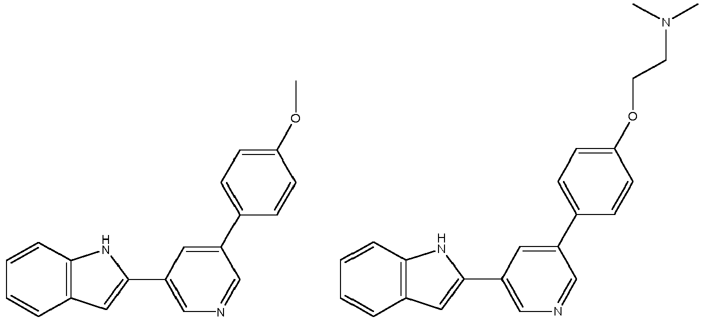 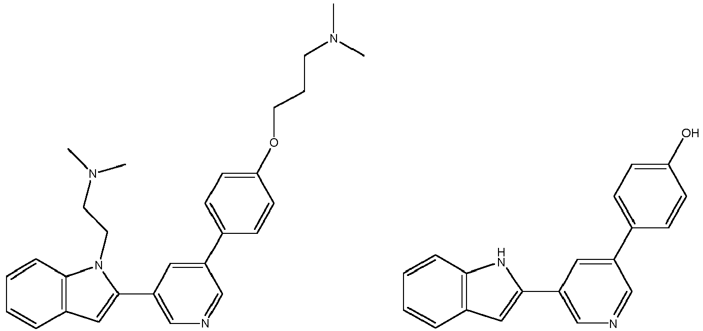 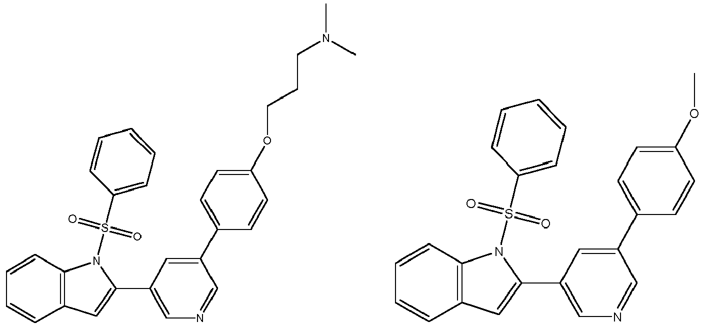 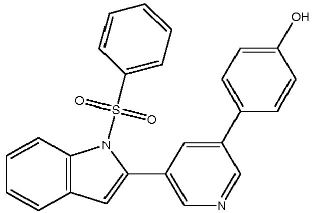 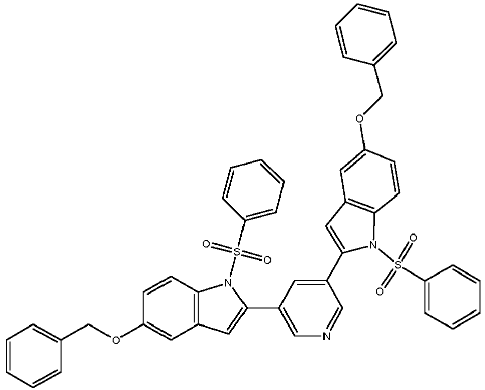 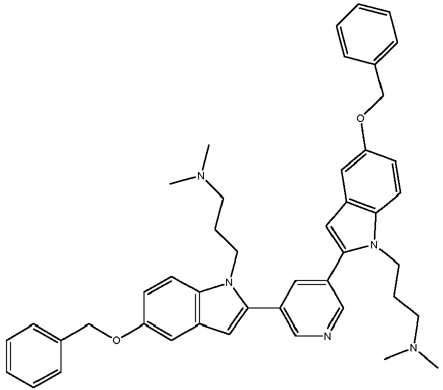 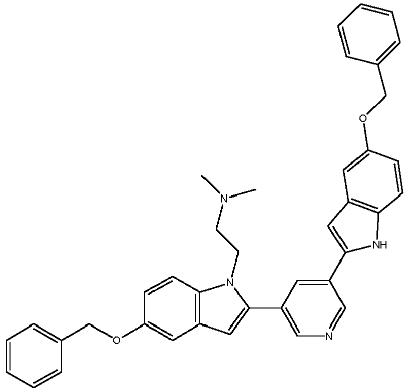 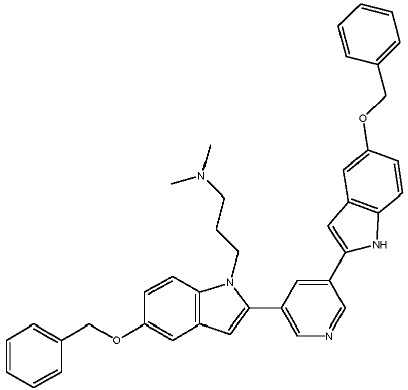 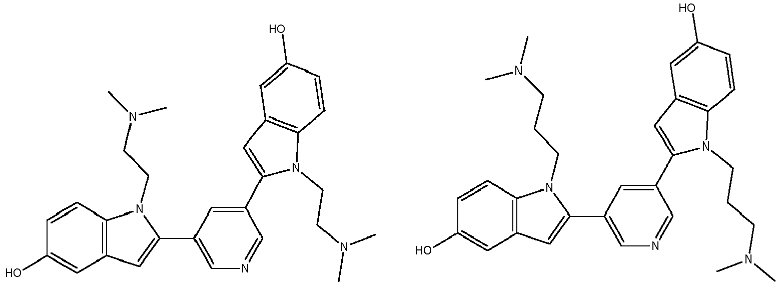 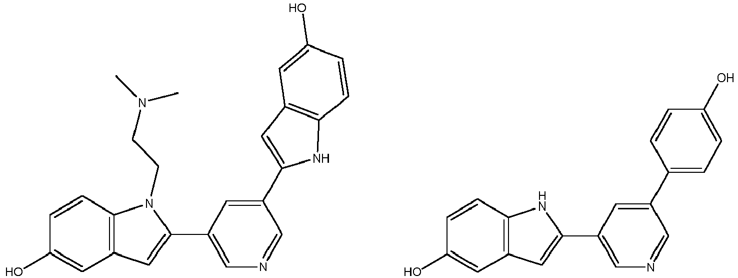 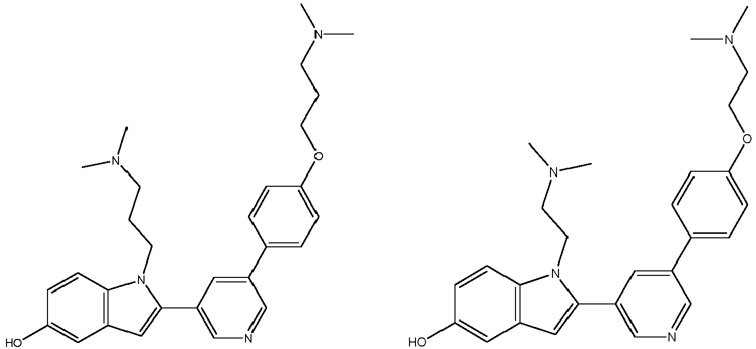 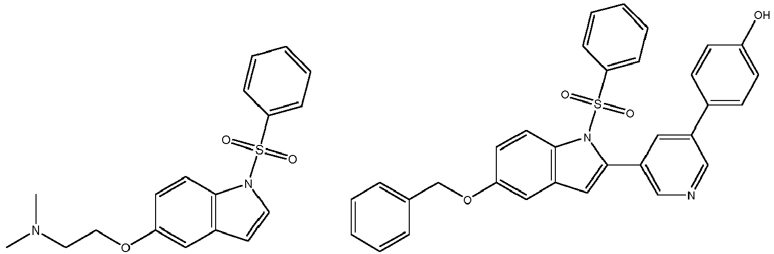 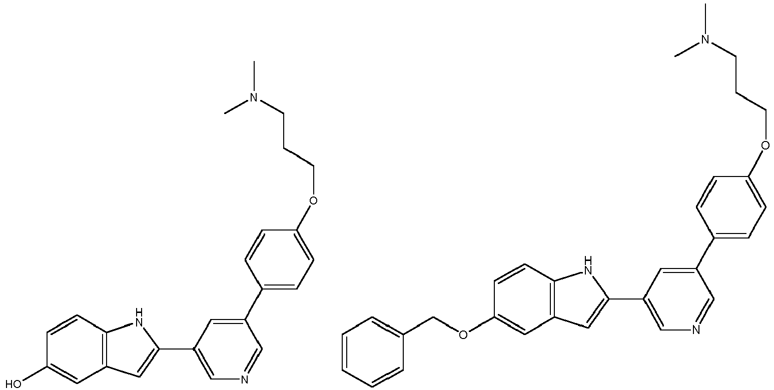 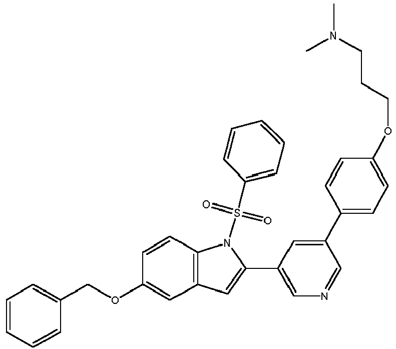 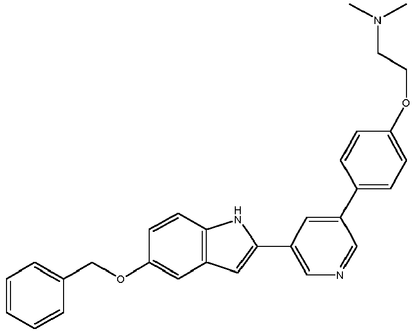 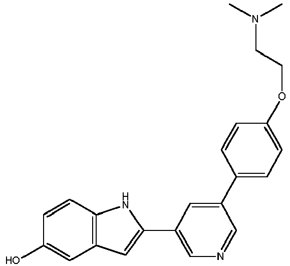 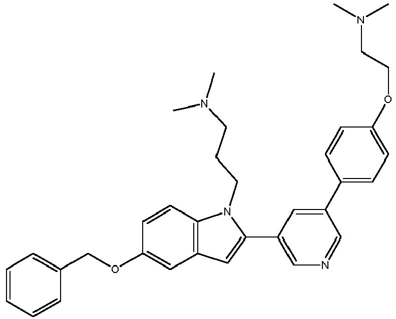 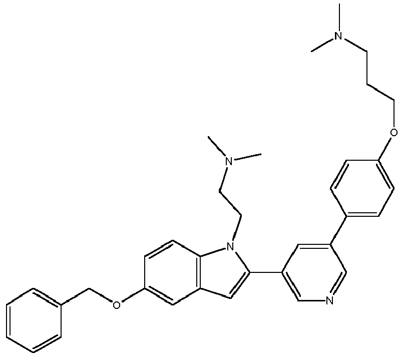 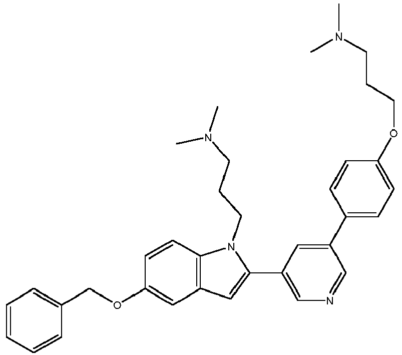 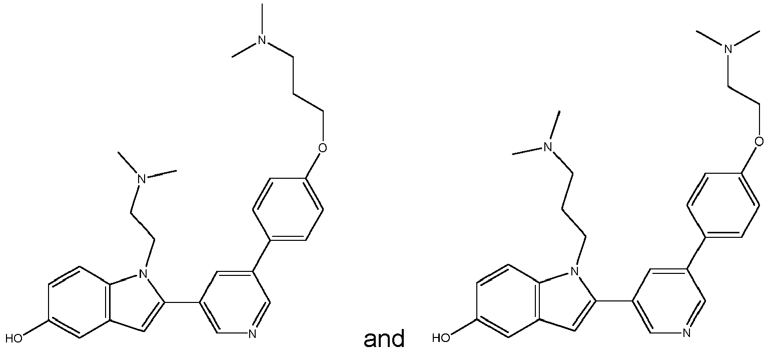 or a pharmaceutically acceptable salt thereof.

4. The compound for use according to anyone of the preceding claims, wherein the compound is in an enteral, parenteral or topical form.

5. The compound for use according to anyone of the preceding claims, wherein the compound is administered in a form chosen among a form for oral administration, a form for topical-transdermal administration, a form for nasal administration, a form for a rectal administration, a subcutaneous form, an intramuscular form, an intratracheal form and an intravenous administration.

6. The compound for use according to claim 4, wherein the compound is administered in a form selected among a solution, a syrup, a suspension, an emulsion, oral drops, an oral effervescent dosage form selected from the group comprising tablets, granules and powders, an oral powder or a multiparticulate system selected from the group comprising beads, granules, mini tablets and micro granules, an orodispersible dosage form selected from the group comprising orodispersible tablets, lyophilised wafers, thin films, a chewable tablet, a tablet, a soft gel capsule and a medical chewing gum, a form for buccal and sublungual routes selected from the group comprising buccal or sublingual tablets, muco adhesive preparation, lozenges, oro-mucosal drops and sprays, ointments, cream, gel, lotion, patch, foam. Alternatively, it may be in a form for a nasal administration, for example selected from the group comprising nasal drops, nasal spray, nasal powder, suppository, hard gelatin capsule.

7. The compound for use according to anyone of the preceding claims, wherein the infection is a SARS-CoV-2 infection.

8. The compound for use according to anyone of the preceding claims, for inhibiting the interaction between Spike and ACE2 proteins.

9. A pharmaceutical composition comprising a compound as defined in claims 1 to 3, for use in the prevention or in the treatment of an infection.

10. The pharmaceutical composition for use as claimed in claim 8, wherein the infection is a SARS-CoV-2 infection.
